# EUROPEAN PATENT APPLICATION

(11) **EP 4 509 133 A1**
(43) Date of publication of application: **19.02.2025**
(21) Application number: 24764216.8
(22) Date of filing: 29.02.2024
(51) Int. Cl.: A61K 38/17, A61K 31/7076, A61P 35/00, A61K 45/06, C07K 14/47

(54) **ADP-RIBOSE-BINDING PEPTIDE FOR PREVENTING OR TREATING CANCER, AND COMBINATION THERAPY OF ADP-RIBOSE**

(30) Priority: 02.03.2023 KR 20230028059; 13.03.2023 KR 20230032728
(71) Applicant: Pearlsinmires Co., Ltd., Seoul 07292 (KR)
(72) Inventor: JEONG, Keun-Yeong, Seoul 03690 (KR); PARK, Min Hee, Siheung-si, Gyeonggi-do 14914 (KR)
(74) Representative: Turner, Craig Robert
(86) International application number: PCT/KR2024/002661
(87) International publication number: WO 2024/181817

(57) **Abstract**

The present disclosure relates to anti-cancer uses utilizing an adenosine diphosphate (ADP)-ribose binding peptide and ADP-ribose. A pharmaceutical composition of the present disclosure accumulates ADP-ribose in cancer cells, thereby disrupting the cellular balance to induce cancer cell death, and having excellent anti-cancer effects, particularly in cancers that are resistant to conventional PRAP inhibitors.

## Description

### [Technical Field]

The present disclosure relates to anti-cancer uses utilizing an adenosine diphosphate (ADP)-ribose binding peptide and ADP-ribose.

### [Background Art]

Poly(ADP-ribose) polymerase (PARP) contains ADP-ribosyltransferases that catalyze the linkage of ADP-ribose, and has been identified as a total of 18 members of the PARP family. The primary role of PARP is to perform essential functions in cell survival processes such as DNA damage repair and chromatin remodeling, transcription, and cell death signaling. Among the various PARP family members, PARP1 was first described as a key enzyme involved in repairing DNA base damage or single-strand breaks (SSBs) caused by harmful stimuli from both inside and outside the cell. PARP1 participates in the repair of DNA base damage by recruiting X-ray cross-complementing group 1 (XRCC1), a scaffold protein in base excision repair (BER), to the site of damage. It is also well known for its pivotal role in the repair of DNA double strand breaks (DSBs) through homologous recombination (HR) and non-homologous end joining (NHEJ) . One of the important biochemical actions of the PARPs of the PARP1, PARP2, PARP3, PARP4, and PARP5 (tankyrases 1 and 2) family in performing its functions is to induce poly(ADP-ribosylation) (PARylation) to synthesize poly(ADP-ribose) (PAR) chains. The majority of PAR synthesis induced by the PARP family is utilized in response to DNA damage, and also regulates, in addition to DNA repair, transcription, apoptosis, chromatin remodeling, antioxidation, inflammation, metabolic regulation, cell cycle regulation, differentiation, proteasomal degradation, RNA processing, and tumor suppressor factors through binding to the surface of numerous intracellular proteins. In other words, PARP's induction of PARylation is involved in the response to cellular stress and, more importantly, it highlights PARP an enzyme that regulates a variety of sophisticated cellular physiological processes. Based on this background, there has been a recent focus on the inhibition of PARP in the field of cancer therapy, and various PARP inhibitors have been developed and utilized in the clinic.

In December 2014, the European Medicines Agency (EMA) and the U.S. FDA approved olaparib as a monotherapy for patients with advanced ovarian cancer with a germline BRCA mutation who have received three or more prior lines of chemotherapy. In addition, Rucaparib was accelerated approval by the U.S. FDA on December 19, 2016 as a treatment for previously treated BRCA-mutant ovarian cancer and obtained full approval in April 2018. Niraparib was approved by the U.S. FDA in March 2017 for epithelial ovarian cancer, fallopian tube cancer, and primary peritoneal cancer. Talazoparib, an inhibitor of PARP1 and PARP2, was approved by the U.S. FDA in 2018 for breast cancer with germline BRCA mutations. These PARP inhibitors have been developed based on synthetic lethality intervention technology and have been recognized as clinically important cancer therapeutic agents as described above. However, recent attention has been drawn to resistance and limitations in the clinical application of PARP inhibitors, highlighting unmet medical needs. There are many reasons for unmet medical needs for cancer treatment with PARP inhibitors, but one of the most well-recognized is the problem of resistance in cancer treatment. During synthetic lethality interventions with PARP inhibitors, cancer cells with microsatellite stable/microsatellite instable-low (MSS/MSI-L) characteristics may develop resistance to PARP inhibitors, and cancer cells with deletion of the P53 gene binding protein 1 (TP53BP1) may develop resistance due to secondary mutations that restore homologous recombination function, posing challenges in overcoming PARP inhibitor resistance. Resistance conditions may create a tumor microenvironment that may lead to increased expression of poly(ADP-ribose) (PAR) due to reactivation of PARP, and the increased PAR plays an important role in the stress overcoming and survival of cancer cells as described above, but when accumulated in excess, may trigger a unique death mechanism called parthanatos. Therefore, its expression is regulated in a highly dynamic manner by poly(ADP-ribose) glycohydrolase (PARG) in cancer cells. Therefore, if it is possible to maintain the synthesized PAR in cancer cells, the parthanatos mechanism is able to induce cancer cell death, offering a potential treatment approach for cancer across various indications and addressing the unmet medical needs caused by resistance to PARP inhibitors.

### [Detailed Description of the Invention]

### [Technical Problem]

The present inventor completed the present disclosure by confirming that the combination of ADP-ribose and ADP-ribose binding peptide has excellent anti-cancer effects, not only on common cancer cells but also on cancer cells resistant to PARP inhibitors.

### [Technical Solution]

An object of the present disclosure is to provide a pharmaceutical composition for preventing or treating cancer comprising: a) an adenosine diphosphate (ADP)-ribose binding peptide or a pharmaceutically acceptable salt thereof; and b) ADP-ribose, a precursor thereof, or a pharmaceutically acceptable salt thereof.

Another object of the present disclosure is to provide a pharmaceutical composition for preventing or treating poly(ADP-ribose) polymerase (PARP) inhibitor-resistant cancer.

Still another object of the present disclosure is to provide a pharmaceutical composition for preventing or treating cancer comprising an ADP-ribose binding peptide or a pharmaceutically acceptable salt thereof as an active ingredient, wherein the ADP-ribose binding peptide is derivatized to have sustained-release properties.

Still another object of the present disclosure is to provide a method of preventing or treating cancer, comprising administering the pharmaceutical composition to an individual in need thereof.

Still another object of the present disclosure is to provide a use of the pharmaceutical composition for use in the prevention or treatment of cancer.

### [Advantageous Effects]

The pharmaceutical composition of the present disclosure may accumulate ADP-ribose in cancer cells, thereby disrupting the cellular balance to induce cancer cell death, and having excellent anti-cancer effects, particularly in cancers that are resistant to conventional PRAP inhibitors.

### [Description of Drawings]

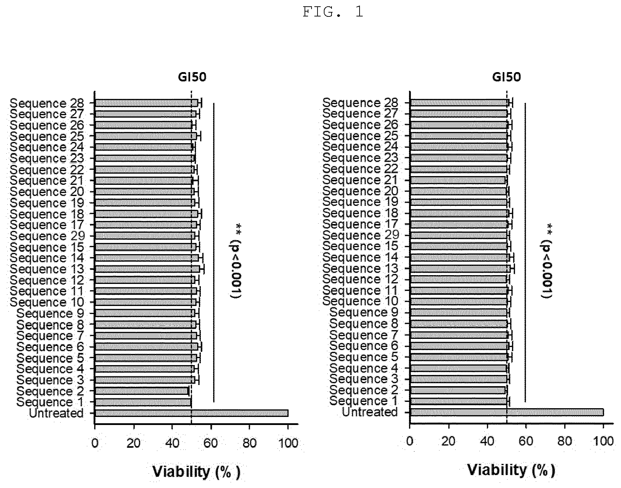

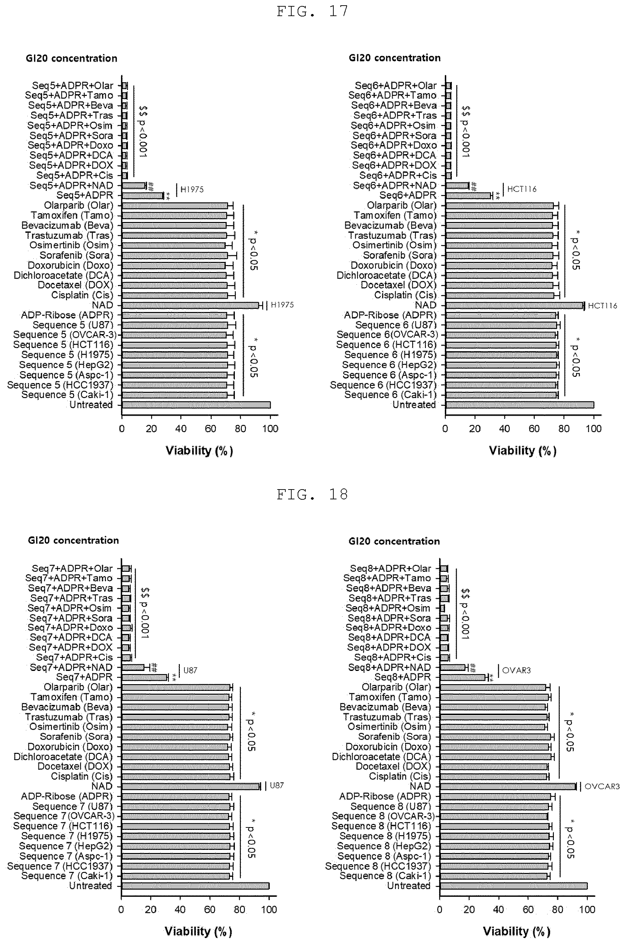

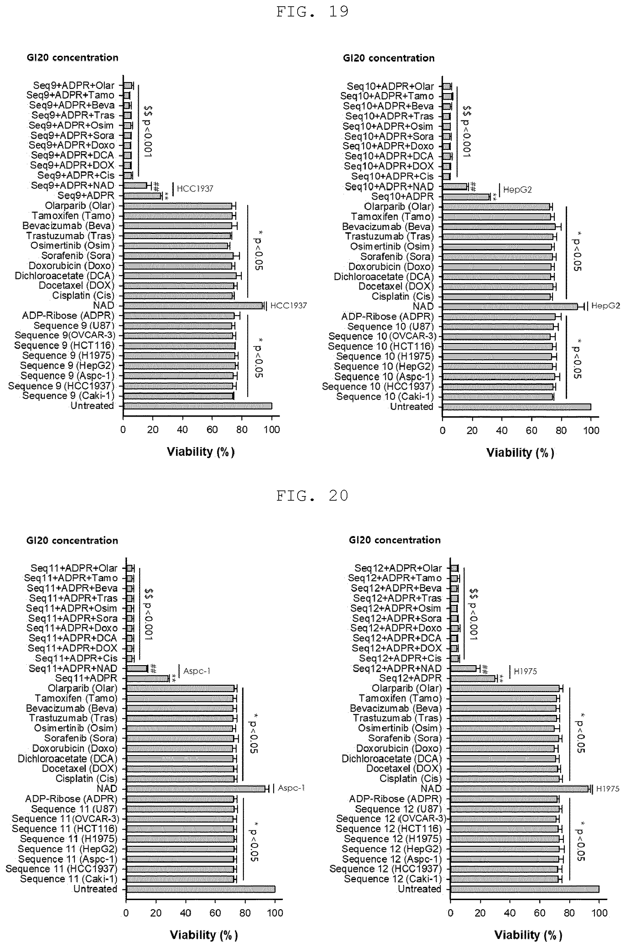

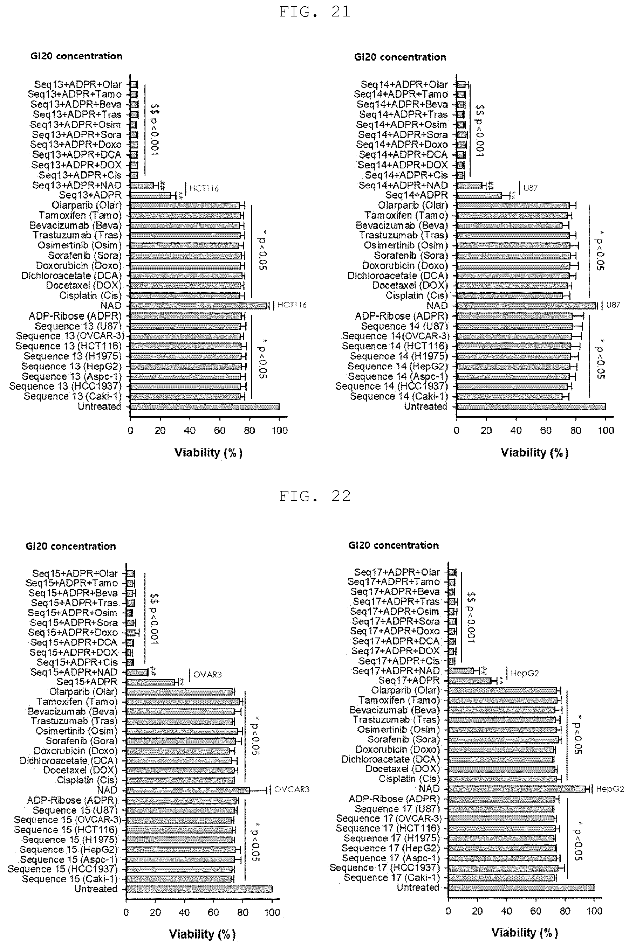

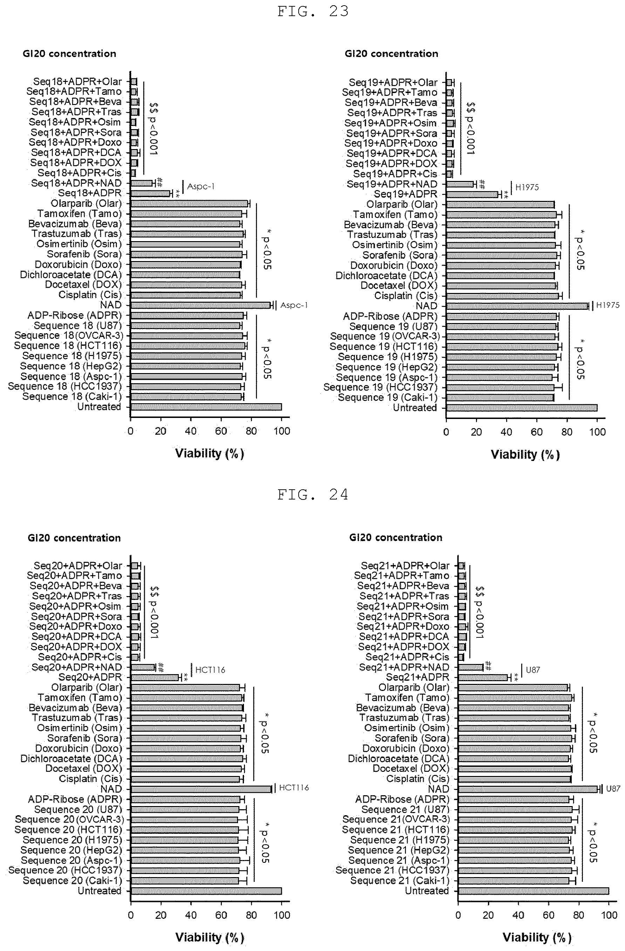

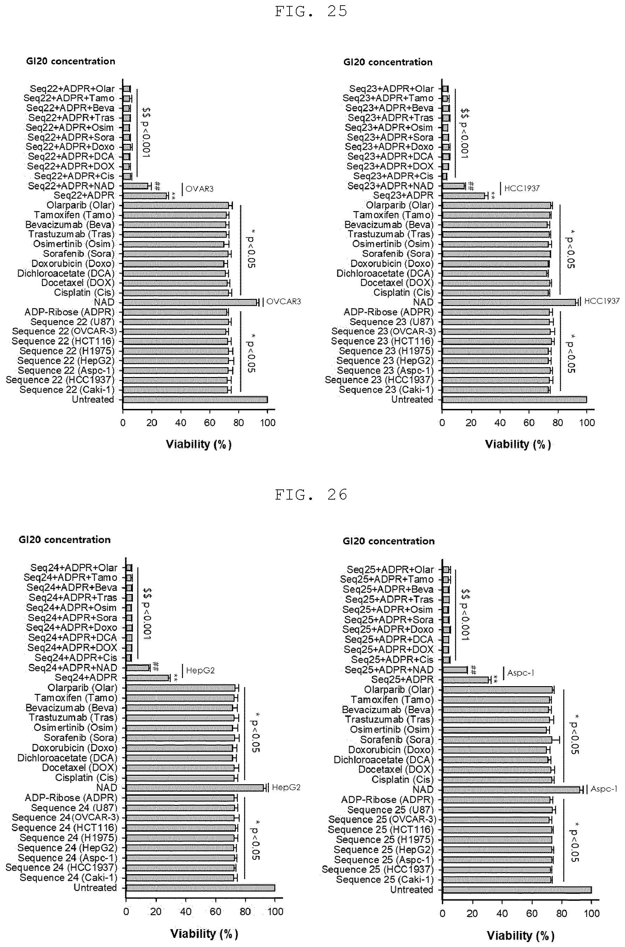

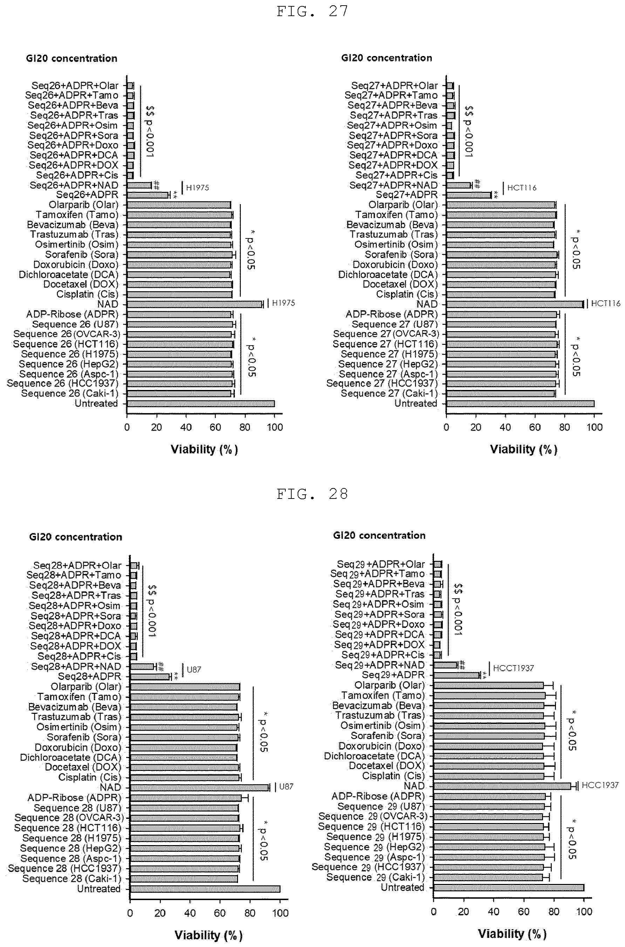

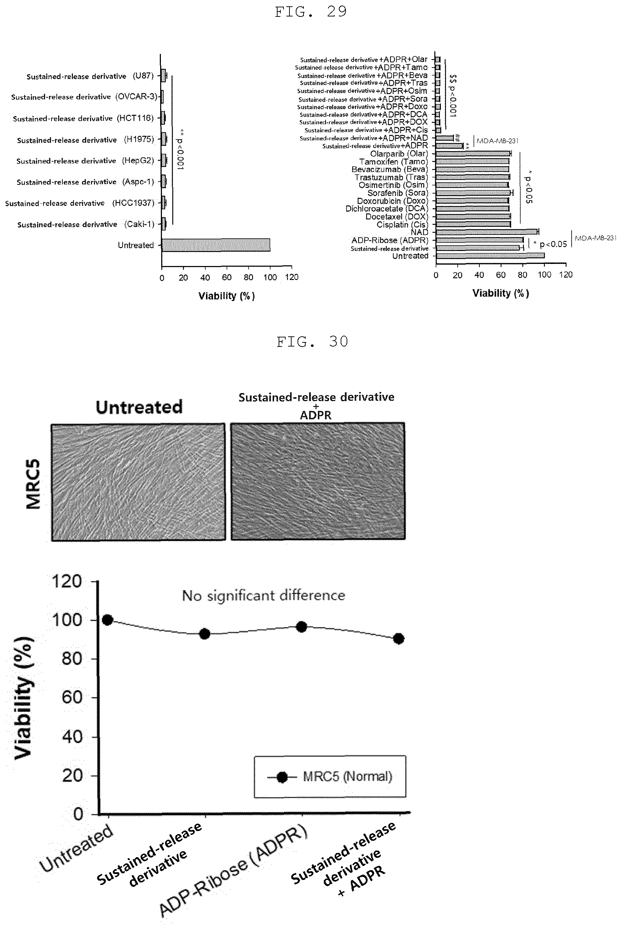

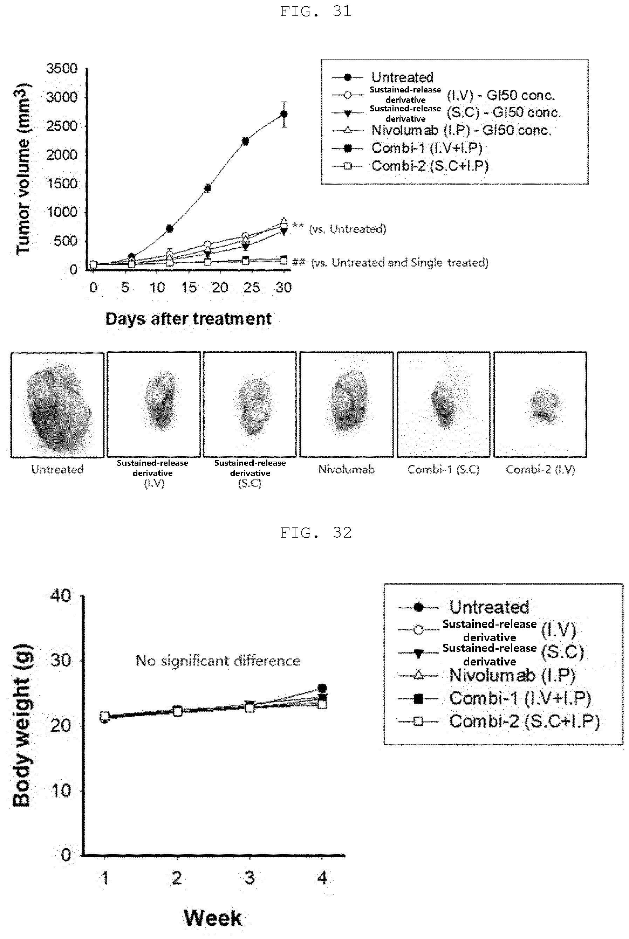

FIG. 1 shows the result of confirming viability and GI50 concentration of each sequence peptide after treatment of pancreatic cancer (Aspc-1) cells and breast cancer (MDA-MB-231) cells with peptides of SEQ ID Nos: 1 to 15 and 17 to 29 alone, wherein the left graph shows results for Aspc-1 cells, and the right graph shows results for MDA-MB-231 cells. ** p < 0.001 vs untreated control.

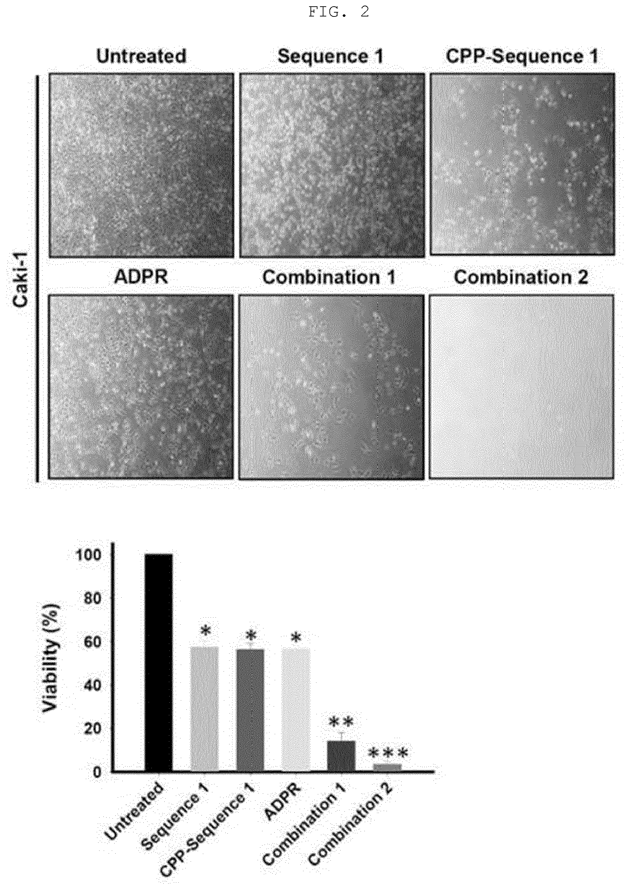

FIG. 2 shows the result of confirming cell viability after treatment of kidney cancer (Caki-1) cells with ADP-ribose and peptides of SEQ ID NO: 1 or SEQ ID NO: 15 each at GI50 concentration, alone or in combination, wherein the top image shows cells, and the bottom graph shows cell viability. * p < 0.001 vs untreated control. ** *p* < 0.001 vs untreated control and single treatment group. *** *p* < 0.001 vs untreated control, single treatment group and Combination 1 group.

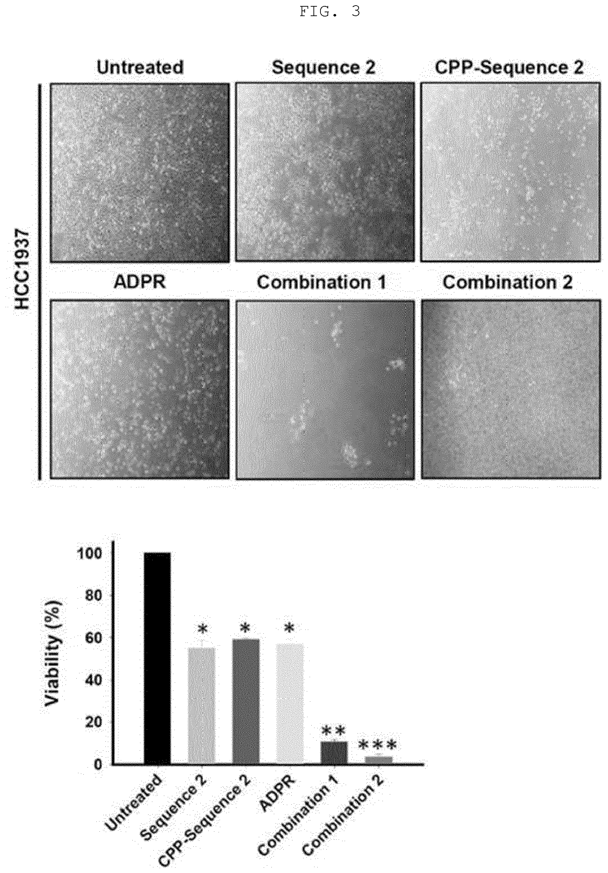

FIG. 3 shows the result of confirming cell viability after treatment of breast cancer (HCC1937) cells with ADP-ribose and peptides of SEQ ID NO: 2 or SEQ ID NO: 16 each at GI50 concentrations, alone or in combination, wherein the top image shows cells, and the bottom graph shows cell viability. * *p* < 0.001 vs untreated control. ** *p* < 0.001 vs untreated control and single treatment group. *** *p* < 0.001 vs untreated control, single treatment group and Combination 1 group.

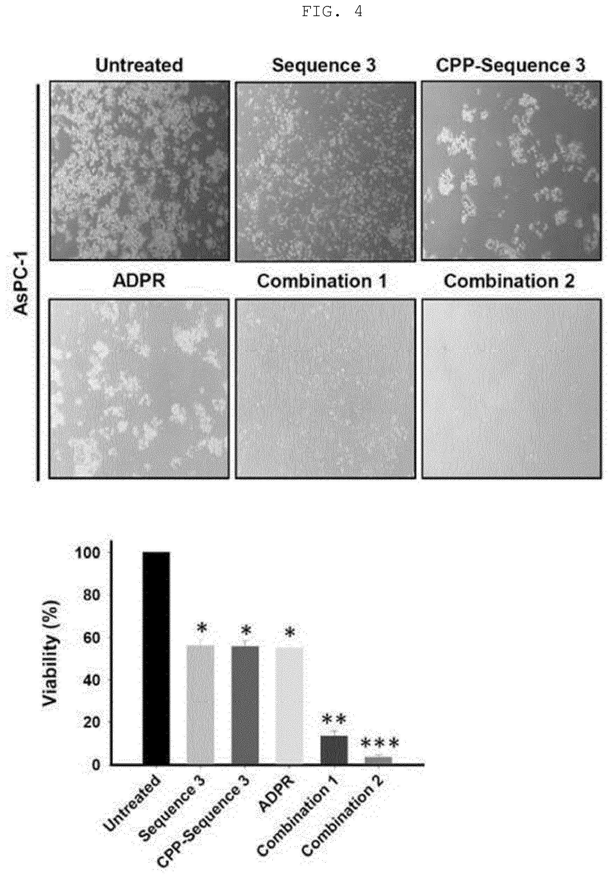

FIG. 4 shows the result of confirming cell viability after treatment of pancreatic cancer (AsPC-1) cells with ADP-ribose and peptides of SEQ ID NO: 3 or SEQ ID NO: 17 each at GI50 concentration, alone or in combination, wherein the top image shows cells, and the bottom graph shows cell viability. * p < 0.001 vs untreated control. ** p < 0.001 vs untreated control and single treatment group. *** p < 0.001 vs untreated control, single treatment group, and Combination 1 group.

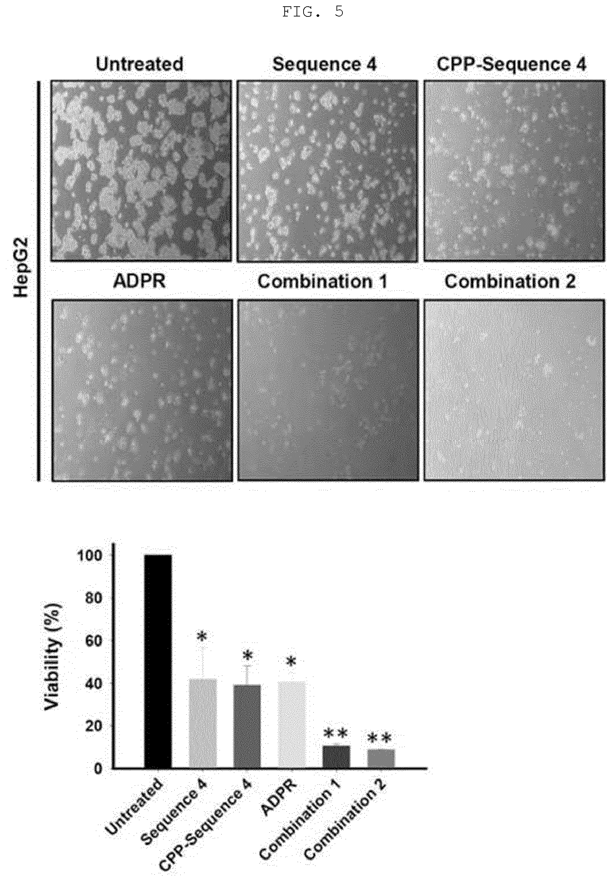

FIG. 5 shows the result of confirming cell viability after treatment of liver cancer (HepG2) cells with ADP-ribose and peptides of SEQ ID NO: 4 or SEQ ID NO: 18 each at GI50 concentration, alone or in combination, wherein the top image shows cells, and the bottom graph shows cell viability. * *p* < 0.001 vs untreated control. ** *p* < 0.001 vs untreated control and single treatment group. *** *p* < 0.001 vs untreated control, single treatment group, and Combination 1 group.

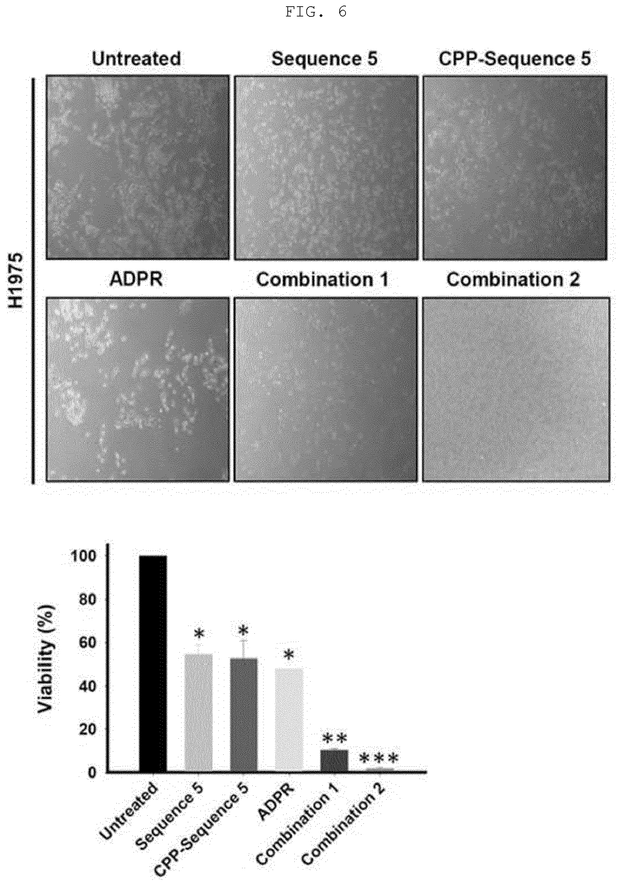

FIG. 6 shows the result of confirming cell viability after treatment of lung cancer (H1975) cells with ADP-ribose and peptides of SEQ ID NO: 5 or SEQ ID NO: 19 each at GI50 concentration, alone or in combination, wherein the top image shows cells, and the bottom graph shows cell viability. * *p* < 0.001 vs untreated control. ** *p* < 0.001 vs untreated control and single treatment group. *** *p* < 0.001 vs untreated control, single treatment group, and Combination 1 group.

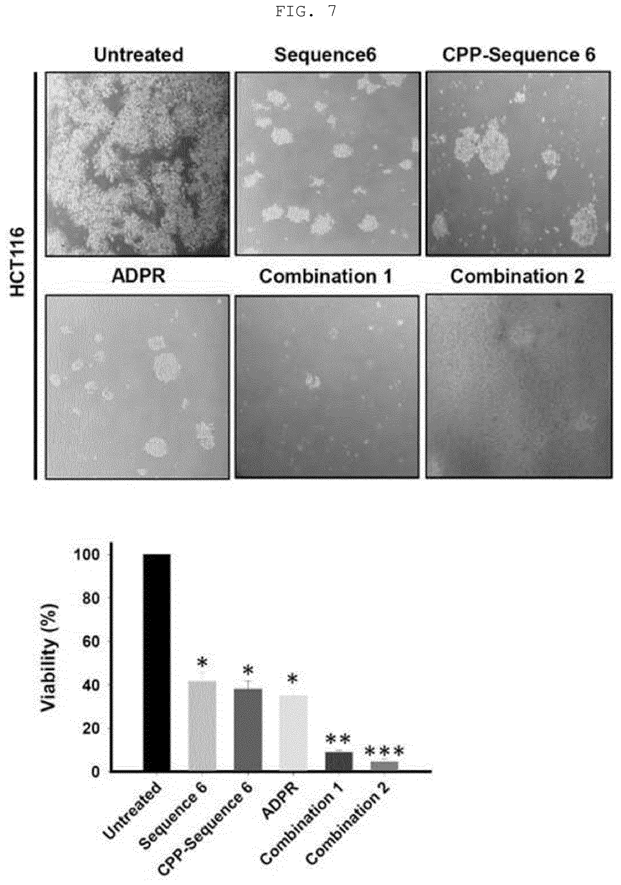

FIG. 7 shows the result of confirming cell viability after treatment of colon cancer (HCT116) cells with ADP-ribose and peptides of SEQ ID NO: 6 or SEQ ID NO: 20 each at GI50 concentration, alone or in combination, wherein the top image shows cells, and the bottom graph shows cell viability. * p < 0.001 vs untreated control. ** *p* < 0.001 vs untreated control and single treatment group. *** *p* < 0.001 vs untreated control, single treatment group, and Combination 1 group.

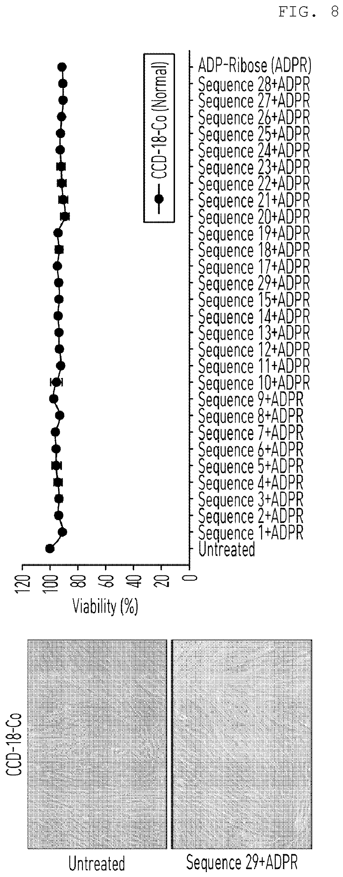

FIG. 8 shows the result of confirming cell viability after treatment of human-derived normal colon (CCD-18-Co) fibroblasts with ADP-ribose alone or in combination with peptides of SEQ ID NOs: 1 to 15 and 17 to 29, wherein the bottom image shows cells, and the top graph shows cell viability.

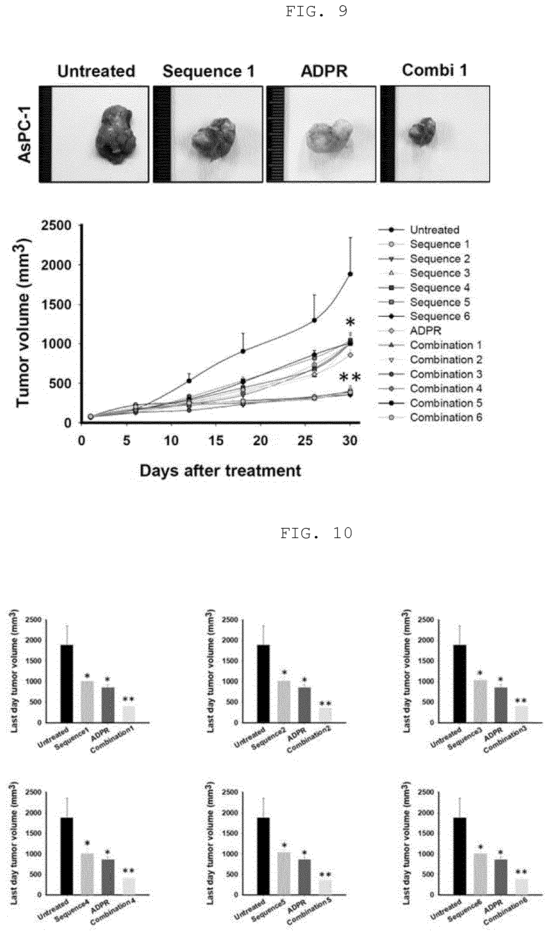

FIG. 9 shows the change in tumor volume over time in a pancreatic cancer (AsPC-1) xenograft animal model after subcutaneous treatment with ADP-ribose and peptides of SEQ ID NOs: 1 to 6 each at GI50 concentration, alone or in combination. * *p* < 0.001 vs untreated control. ** *p* < 0.001 vs untreated control and single treatment group.
FIG. 10 is a graph showing the tumor volume in a pancreatic cancer (AsPC-1) xenograft animal model at the end of the test after subcutaneous treatment with ADP-ribose and peptides of SEQ ID NOs: 1 to 6 each at GI50 concentration, alone or in combination. * *p* < 0.001 vs untreated control. ** *p* < 0.001 vs untreated control and single treatment group.

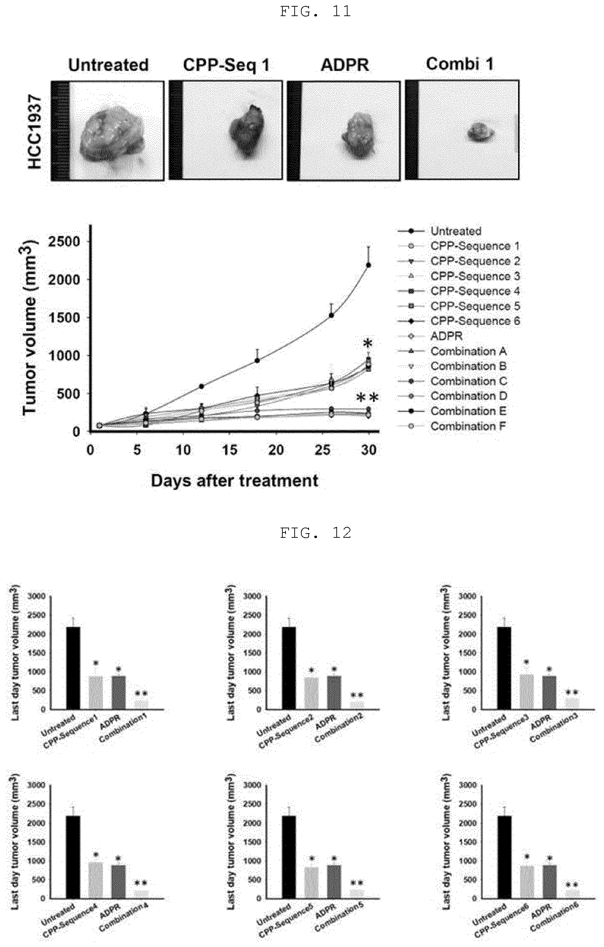

FIG. 11 shows the change in tumor volume over time in a breast cancer (HCC1937) xenograft animal model after subcutaneous treatment with ADP-ribose and peptides of SEQ ID NOs: 15 to 20 each at GI50 concentration, alone or in combination. * *p* < 0.001 vs untreated control. ** *p* < 0.001 vs untreated control and single treatment group.
FIG. 12 is a graph showing the tumor volume in a breast cancer (HCC1937) xenograft animal model at the end of the test after oral treatment with ADP-ribose and peptides of SEQ ID NOs: 15 to 20 each at GI50 concentration, alone or in combination. * *p* < 0.001 vs untreated control. ** *p* < 0.001 vs untreated control and single treatment group.

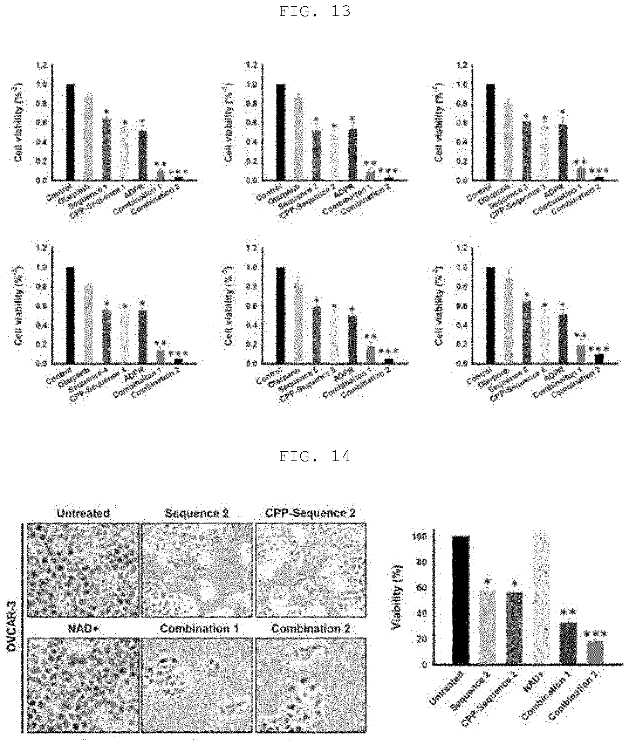

FIG. 13 shows the cell viability of after treatment of PARP inhibitor-resistant breast cancer (HCC1937) cells with olaparib alone, ADP-ribose and peptides of SEQ ID NOs: 1 to 6 or peptides of SEQ ID NOs: 15 to 20 each at GI50 concentration, alone or in combination. * *p* < 0.001 vs untreated control and olaparib-treated group. ** *p* < 0.001 vs untreated control and single treatment group. *** *p* < 0.001 vs untreated control, single treatment group and Combination 1 group.
FIG. 14 shows the result of confirming cell viability after treatment of ovarian cancer (OVCAR-3) cells with NAD⁺ and peptides of SEQ ID NO: 2 or SEQ ID NO: 16 each at GI50 concentration, alone or in combination, wherein the left image shows cells, and the right graph shows cell viability. * *p* < 0.001 vs untreated control. ** *p* < 0.001 vs untreated control and single treatment group. *** *p* < 0.001 vs untreated control, single treatment group, and Combination 1 group.

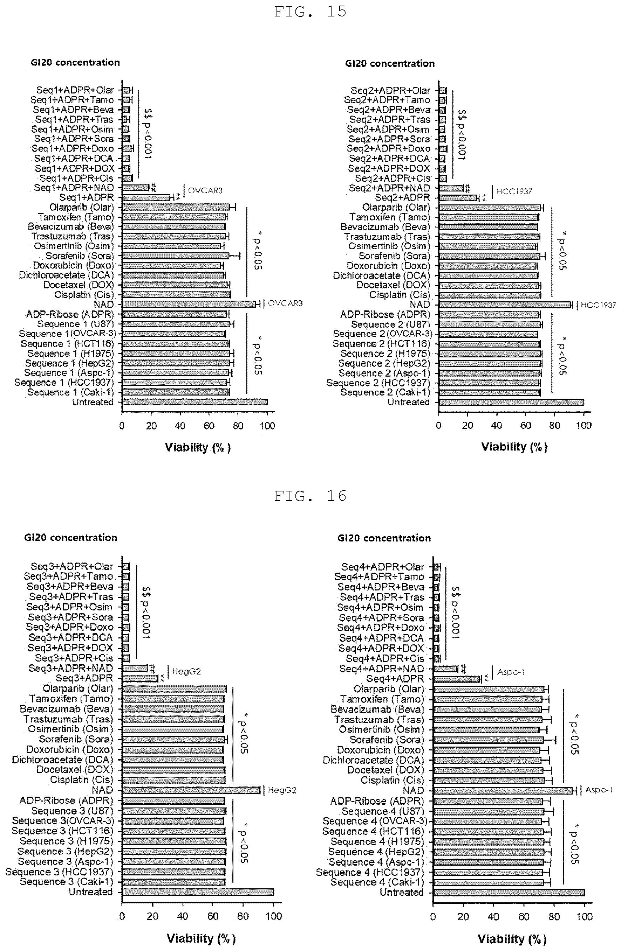

FIG. 15 shows the result of confirming cell viability after treatment of various cancer cells, including ovarian cancer (OVCAR-3) and breast cancer (HCC1937) cells, with peptides of SEQ ID NO: 1 or 2 at GI20 concentration, alone or in combination with ADP-ribose, NAD⁺, and/or various anti-cancer drugs, wherein the left graph shows results for the peptide with SEQ ID NO: 1, and the right graph shows results for the peptide with SEQ ID NO: 2. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 16 shows the result of confirming cell viability after treatment of various cancer cells, including liver cancer (HepG2) and pancreatic cancer (Aspc-1) cells, with peptides of SEQ ID NO: 3 or 4 each at GI20 concentration, alone or in combination with ADP-ribose, NAD⁺, and/or various anti-cancer drugs, wherein the left graph shows results for the peptide with SEQ ID NO: 3, and the right graph shows results for the peptide with SEQ ID NO: 4. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 17 shows the result of confirming cell viability after treatment of various cancer cells, including lung cancer (H1975) and colon cancer (HCT116) cells, with peptides of SEQ ID NO: 5 or 6 at GI20 concentration, alone or in combination with ADP-ribose, NAD⁺, and/or various anti-cancer drugs, wherein the left graph shows results for the peptide with SEQ ID NO: 5, and the right graph shows results for the peptide with SEQ ID NO: 6. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 18 shows the result of confirming cell viability after treatment of various cancer cells, including brain cancer (U87) and ovarian cancer (OVCAR-3) cells, with peptides of SEQ ID NO: 7 or 8 at GI20 concentration, alone or in combination with ADP-ribose, NAD⁺, and/or various anti-cancer drugs, wherein the left graph shows results for the peptide with SEQ ID NO: 7, and the right graph shows results for the peptide with SEQ ID NO: 8. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 19 shows the result of confirming cell viability after treatment of various cancer cells, including breast cancer (HCC1937) and liver cancer (HepG2) cells, with peptides of SEQ ID NO: 9 or 10 at GI20 concentration, alone or in combination with ADP-ribose, NAD⁺, and/or various anti-cancer drugs, wherein the left graph shows results for the peptide with SEQ ID NO: 9, and the right graph shows results for the peptide with SEQ ID NO: 10. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 20 shows the result of confirming cell viability after treatment of various cancer cells, including pancreatic cancer (Aspc-1) and lung cancer (H1975) cells, with peptides of SEQ ID NO: 11 or 12 at GI20 concentration, alone or in combination with ADP-ribose, NAD⁺, and/or various anti-cancer drugs, wherein the left graph shows results for the peptide with SEQ ID NO: 11, and the right graph shows results for the peptide with SEQ ID NO: 12. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 21 shows the result of confirming cell viability after treatment of various cancer cells, including colon cancer (HCT116) and brain cancer (U87) cells, with peptides of SEQ ID NO: 13 or 14 at GI20 concentration, alone or in combination with ADP-ribose, NAD⁺, and/or various anti-cancer drugs, wherein the left graph shows results for the peptide with SEQ ID NO: 13, and the right graph shows results for the peptide with SEQ ID NO: 14. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 22 shows the result of confirming cell viability after treatment of various cancer cells, including ovarian cancer (OVCAR-3) and liver cancer (HepG2) cells, with peptides of SEQ ID NO: 15 or 17 at GI20 concentration, alone or in combination with ADP-ribose, NAD⁺, and/or various anti-cancer drugs, wherein the left graph shows results for the peptide with SEQ ID NO: 15, and the right graph shows results for the peptide with SEQ ID NO: 17. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 23 shows the result of confirming cell viability after treatment of various cancer cells, including pancreatic cancer (Aspc-1) and lung cancer (H1975) cells, with peptides of SEQ ID NO: 18 or 19 at GI20 concentration, alone or in combination with ADP-ribose, NAD⁺, and/or various anti-cancer drugs, wherein the left graph shows results for the peptide with SEQ ID NO: 18, and the right graph shows results for the peptide with SEQ ID NO: 19. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 24 shows the result of confirming cell viability after treatment of various cancer cells, including colon cancer (HCT116) and brain cancer (U87) cells, with peptides of SEQ ID NO: 20 or 21 at GI20 concentration, alone or in combination with ADP-ribose, NAD⁺, and/or various anti-cancer drugs, wherein the left graph shows results for the peptide with SEQ ID NO: 20, and the right graph shows results for the peptide with SEQ ID NO: 21. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 25 shows the result of confirming cell viability after treatment of various cancer cells, including ovarian cancer (OVCAR-3) and breast cancer (HCC1937) cells, with peptides of SEQ ID NO: 22 or 23 at GI20 concentrations, alone or in combination with ADP-ribose, NAD⁺, and/or various anti-cancer drugs, wherein the left graph shows results for the peptide with SEQ ID NO: 22, and the right graph shows results for the peptide with SEQ ID NO: 23. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 26 shows the result of confirming cell viability after treatment of various cancer cells, including liver cancer (HepG2) and pancreatic cancer (Aspc-1) cells, with peptides of SEQ ID NO: 24 or 25 each at GI20 concentration, alone or in combination with ADP-ribose, NAD⁺, and/or various anti-cancer drugs, wherein the left graph shows results for the peptide with SEQ ID NO: 24, and the right graph shows results for the peptide with SEQ ID NO: 25. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 27 shows the result of confirming cell viability after treatment of various cancer cells, including lung cancer (H1975) and colon cancer (HCT116) cells, with peptides of SEQ ID NO: 26 or 27 at GI20 concentration, alone or in combination with ADP-ribose, NAD⁺, and/or various anti-cancer drugs, wherein the left graph shows results for the peptide with SEQ ID NO: 26, and the right graph shows results for the peptide with SEQ ID NO: 27. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 28 shows the result of confirming cell viability after treatment of various cancer cells, including brain cancer (U87) and breast cancer (HCC1937) cells, with peptides of SEQ ID NO: 28 or 29 at GI20 concentration, alone or in combination with ADP-ribose, NAD⁺, and/or various anti-cancer drugs, wherein the left graph shows results for the peptide with SEQ ID NO: 28, and the right graph shows results for the peptide with SEQ ID NO: 29. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 29 shows the result of confirming cell viability after treatment of various cancer cells with sustained-release derivative at GI20 concentration, which was prepared by PEGylating the peptide of SEQ ID NO: 29 and binding a fatty acid, alone or in combination with ADP-ribose, NAD⁺, and/or various anti-cancer drugs, wherein the left graph shows results for the sustained-release derivative alone, and the right graph shows results of the combination treatment. * *p* < 0.05 vs untreated control. ** *p* < 0.001 vs other groups except for the anti-cancer drug combination group and the ADP-ribose and NAD⁺ combination group. ## *p* < 0.001 vs other groups except for the anti-cancer drug combination group. $$ *p* < 0.001 vs other groups.
FIG. 30 shows the result of confirming cell viability after treatment of human-derived normal lung (MRC5) fibroblasts with ADP-ribose and sustained-release derivative alone or in combination, wherein the top image shows cells, and the bottom graph shows cell viability.
FIG. 31 shows tumor volume changes over time in a colon cancer (MC38) xenograft animal model after intravenous, subcutaneous, or intraperitoneal treatment with the immunotherapeutic agent Nivolumab and sustained-release derivative each at GI50 concentration, alone or in combination. ** *p* < 0.001 vs untreated control. ## *p* < 0.001 vs untreated control and single treatment group.
FIG. 32 shows body weight changes over time in a colon cancer (MC38) xenograft animal model after intravenous, subcutaneous, or intraperitoneal treatment with the immunotherapeutic agent Nivolumab and sustained-release derivative, alone or in combination.

### [Best Mode]

The present disclosure will be described in detail as follows. Meanwhile, each description and embodiment disclosed in the present disclosure may be applied to each of the other descriptions and embodiments. In other words, all combinations of various elements disclosed in the present disclosure fall within the scope of the present disclosure. In addition, it cannot be considered that the scope of the present disclosure is limited by specific descriptions described below.

An object of the present invention is to provide a pharmaceutical composition for preventing or treating cancer comprising: a) an adenosine diphosphate (ADP)-ribose binding peptide or a pharmaceutically acceptable salt thereof; and b) ADP-ribose, a precursor thereof, or a pharmaceutically acceptable salt thereof.

In another general aspect, there is provided a method of preventing or treating cancer, comprising administering the pharmaceutical composition to an individual in need thereof.

In still another general aspect, there is provided a use of the pharmaceutical composition for use in the prevention or treatment of cancer.

As used herein, the term "ADP-ribose binding peptide" refers to any peptide having the activity of binding to ADP-ribose or an ADP-ribose polymer and inhibiting its degradation. In the present disclosure, the peptide may include not simply a peptide formed by peptide bonds between the amino acids comprising the peptide, but also all forms of peptide analogs, derivatives, and the like, which have some modifications to improve properties such as stability, efficacy, and the like in the context of protein medicaments.

In an embodiment, the term "ADP-ribose binding peptide" used herein may consist of any one amino acid sequence selected from the group consisting of SEQ ID NO: 1 to SEQ ID NO: 14. The peptides of SEQ ID Nos: 1 to 14 above are all derived from the WWE domain. The WWE domain is a globular domain that is conserved in a number of proteins, including deltex, Trip12, and poly-ADP-ribose polymerase homologs, and is named after the most conserved residue within the domain (L. Aravind, TRENDS in Biochemical Sciences, 2001, 26(5): 273). In some proteins with the WWE domain, it is known that an intra-domain ADP-ribose binding motif is present in the domain. The WWE domain of intracellular enzymes has been reported to bind primarily to ADP-ribose and induce its degradation. In other words, the binding of certain enzymes to PAR or ADPR via WWE domain may be seen as having a major purpose for cancer cell survival (PNAS August 23, 2011 108 (34) 14103-14108).

Meanwhile, the function of the WWE domain in proteins as described above has been reported before, but it has not been reported that when a part of the WWE domain is isolated and prepared as a peptide as in the present disclosure and treated to cells, ADP-ribose degradation is inhibited, intracellular ADP-ribose is accumulated, which is contrary to the above, and as a result, excellent anti-cancer effects are achieved.

The ADP-ribose binding peptide may encompass all within the scope of the present disclosure, including not only peptides comprising the amino acid sequence of any one of SEQ ID NOs: 1 to 14, but also sequences having one or more amino acids added, substituted or deleted from these sequences, provided that they fall within the equivalent range. For example, it is apparent to include, within the scope of the present disclosure, any peptide having at least 80%, 90%, 95%, 97%, or 99% homology to a peptide having an amino acid sequence of any one of SEQ ID NOs: 1 to 14 of the present disclosure, and exhibiting efficacy, i.e., ADP-ribose binding activity and anti-cancer activity corresponding to the peptide consists of amino acid sequence of any one of SEQ ID NOs: 1 to 14, even if some of the amino acid sequences of SEQ ID NOs: 1 to 14 have some additions, substitutions or deletions.

In addition, when exhibiting activity corresponding to the peptide comprising the amino acid sequence of any one of SEQ ID NOs 1 to 14, naturally occurring mutations or nonsensical sequence additions before or after the amino acid sequence, or silent mutations thereof, may be also included within the scope of the present disclosure.

As used herein, the term "homology" means the degree of match to a given amino acid sequence or base sequence and may be expressed as a percentage. In the present disclosure, a homologous sequence having the same or similar activity to the given amino acid sequence or base sequence is denoted as "% homology". For example, homology may be confirmed by using standard software, specifically BLAST 2.0, which calculates parameters such as score, identity, and similarity, etc., or by comparing sequences through Southern hybridization experiments under defined and stringent conditions, wherein the appropriate hybridization conditions defined are within the scope of the art and may be determined by methods well known to those skilled in the art (see, e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York) .

Further, the peptides of the present disclosure may include modifications, such as chemical derivatization, in one or more of the amino acids comprising the peptide to further improve desired properties, and it will be apparent to those skilled in the art that these modifications also fall within the scope of the present disclosure, as long as they have anti-cancer activity equivalent to the peptides of the present disclosure. The derivatization may include, but is not limited to, acetylation, hydroxylation, methylation, amidation, PEGylation; or the addition of fatty acids, carbohydrates, lipid components, cofactors, and the like.

In one specific embodiment of the present disclosure, the peptide may be further utilized in a form that is fused with a cell-penetrating peptide to increase cell permeability. In other words, the ADP-ribose binding peptide may further comprise a cell-penetrating peptide at the N-terminus, C-terminus, or both termini. Here, a linker may be further comprised between the ADP-ribose binding peptide and the cell-penetrating peptide, which may be performed as appropriate by those skilled in the art.

As used herein, the term "cell-penetrating peptide" refers to a peptide having properties capable of facilitating the intracellular uptake/absorption of various substances, including nanoparticles, compounds, DNA, proteins, etc. Specifically, the cell-penetrating peptide may be, but is not limited to, TAT, buforin, maurocalcine, penetratin, poly-arginine-derived peptides, Antennapedia, Transportan, VP22, Hph-1, poly-arginine R11(R9), Pep-1, HP4, LAH4, Vetofusin-1, signal sequence-based peptides, or amphipathic peptides and may be suitably selected by those skilled in the art as long as they are capable of promoting intracellular movement of the ADP-ribose binding peptide of the present disclosure. In a specific embodiment, the ADP-ribose binding peptide comprising the cell-penetrating peptide may be a peptide comprising an amino acid sequence of any one of SEQ ID NO: 15 to SEQ ID NO: 29, but is not limited thereto.

Further, those skilled in the art may modify the ADP-ribose binding peptide of the present disclosure as appropriate for application depending on the type of cell-penetrating peptide being used. In other words, the ADP-ribose binding peptide of the present disclosure is not limited to the amino acid sequences presented herein, even when fused to a cell-penetrating peptide, and may be used with additions/substitutions/removals of amino acid sequences in a form suitable for the application of the cell-penetrating peptide within a range that is apparent to those skilled in the art, i.e., within the equivalent range.

In addition, the ADP-ribose binding peptide of the present disclosure may be used with reagents known in the art to increase cell permeability, enabling to deliver proteins to cells, or improve delivery efficiency. The above reagents may include commercially available reagents such as Chariot^{™} (Active motif, Cat. 30025), Xflect^{™} (Takara, Cat. 631324), Pierce^{™} (ThermoFisher Scientific, Cat. 89850), ProteoJuice^{™} (Merck, Cat. 71281), and PULSin^{™} (Poylplus transfection) as well as other non-commercial reagents, and are not specifically limited to these examples as long as they are capable of delivering the ADP-ribose binding peptide of the present disclosure to cells.

In a specific embodiment of the present disclosure, the ADP-ribose binding peptide may further comprise a cancer-specific peptide at the N-terminus, C-terminus, or both termini. Depending on the desired object, the anti-cancer effect may be further enhanced in vivo by adding peptide sequences specific to the target cancer. As used herein, the term "cancer-specific peptide" refers to a peptide capable of specifically recognizing a protein expressed in a cancer cell. The protein expressed in the cancer cell may be, but is not limited to, cancer-specific receptors such as Integrin αvβ3, EGFR, HER, SSTR2, GnRH-R, Bn receptor, VIP receptor, NTSR1, CCK2R, MC1R, hY1R, and the like. The cancer-specific peptide may be added with, for example, a KCCYSL sequence that recognizes HER2 expression when targeting HER2-positive cancers, a YHWYGYTPQNVI sequence that recognizes EGFR expression when targeting EGFR-positive cancers, or an RRPYIL sequence that recognizes NTSR1 expression when targeting NTSR1-positive cancers, but is not specifically limited thereto.

As used herein, the term "ADP-ribose" is the compound represented by the following Chemical Formula 1:

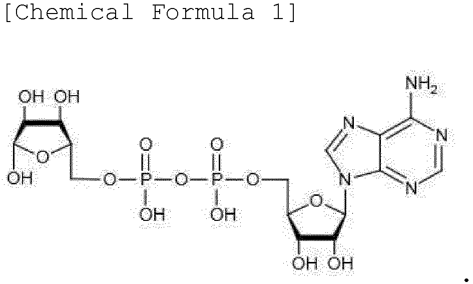

A method for preparing ADP-ribose is known in the art to which the present disclosure pertains. In an embodiment, ADP-ribose is capable of being synthesized via hydrolysis of nicotinamide adenine dinucleotide (NAD⁺) in the presence of an alkaline base. In addition, ADP-ribose may be isolated in the form of a monovalent or divalent salt of the metal ion of the corresponding base. Alternatively, ADP-ribose is commercially available as a purified raw material (CAS Number: 68414-18-6).

As used herein, the term "precursor of ADP-ribose" refers to a substance capable of being converted to ADP-ribose in vivo, such as, but not limited to, NAD⁺, cADPR, poly-ADPR, nicotinic acid, nicotinamide, and nicotinamide riboside.

As used herein, the term "NAD⁺" refers to the compound represented by the following Chemical Formula 2, which is intended to include both the isolated NAD⁺ and its electron-reduced form, NADH:

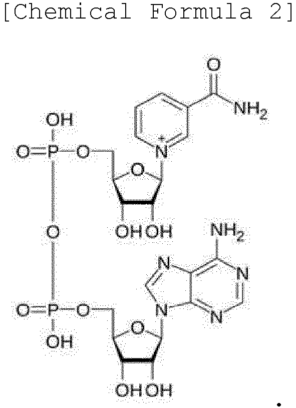

NAD⁺ is the most abundant coenzyme in living organisms involved in redox reactions and acts as a coenzyme for many dehydrogenases, oxidizing hydrogen from various substrates as a hydrogen acceptor in redox reactions such as glycolysis and fermentation. NAD⁺ is a precursor synthesized into ADP-ribose by PARP within cells, and may produce various forms of ADP-ribose through the pathway shown in Reaction Scheme 1 below:

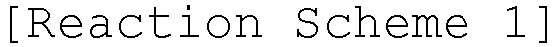

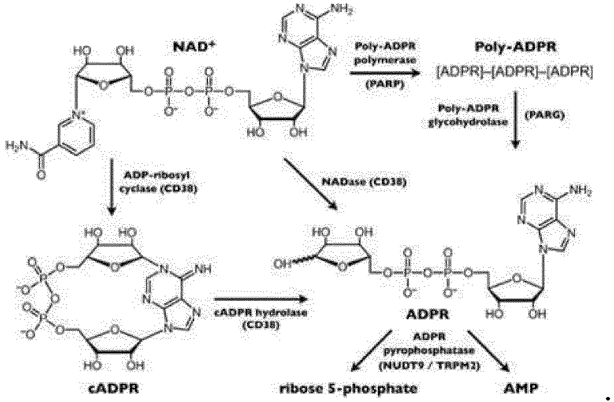

In the present disclosure, the pharmaceutically acceptable salt refers to salts commonly used in the pharmaceutical industry, and may include, for example, salts of inorganic ions including sodium, potassium, calcium, magnesium, lithium, copper, manganese, zinc, iron, and the like, and salts of inorganic acids such as hydrochloric acid, phosphoric acid, and sulfuric acid, and in addition thereto, may include salts of organic acids such as ascorbic acid, citric acid, tartaric acid, lactic acid, maleic acid, malonic acid, fumaric acid, glycolic acid, succinic acid, propionic acid, acetic acid, orotate acid, acetylsalicylic acid, and the like, and salts of amino acids such as lysine, arginine, guanidine, and the like. Further, organic ion salts such as tetramethyl ammonium, tetraethyl ammonium, tetrapropyl ammonium, tetrabutyl ammonium, benzyl trimethyl ammonium, and benzethonium capable of being used in pharmaceutical reactions, purification, and separation processes, may be included. However, the types of salts meant in the present disclosure are not limited by these listed salts. Preferably, the pharmaceutically acceptable salt of ADP-ribose in the present disclosure is not a lithium salt (including mono- and di-lithium salts) of ADP-ribose.

As used herein, the term "cancer" refers to a disease related to the regulation of cell death, which is caused by excessive cell proliferation when the normal cell death balance is broken. In the present disclosure, the cancer may include both malignant tumors and benign tumors, and for example, may be brain cancer, head and neck cancer, lung cancer, breast cancer, thymoma, esophageal cancer, colon cancer, liver cancer, stomach cancer, pancreatic cancer, biliary tract cancer, kidney cancer, bladder cancer, prostate cancer, testicular cancer, germ cell tumor, ovarian cancer, cervical cancer, endometrial cancer, colorectal cancer, lymphoma, acute leukemia, chronic leukemia, multiple myeloma, sarcoma, malignant melanoma, or skin cancer, but the types of cancer of the present disclosure are not limited thereto. The composition for preventing or treating cancer of the present disclosure may have a therapeutic effect on all cancers in which cell death may be caused by the accumulation of intracellular ADP-ribose.

As an example, the cancer may be solid cancer such as kidney cancer, breast cancer, pancreatic cancer, liver cancer, lung cancer, or colon cancer, but is not limited thereto.

In an embodiment of the present disclosure, the cancer may be a poly (ADP-ribose) polymerase (PARP) inhibitor-resistant cancer.

As used herein, the term "PARP inhibitor" refers to an anti-cancer therapeutic agent capable of treating cancer by inhibiting PARP, a key enzyme in the cleavage of single strands of DNA that performs essential functions in cell survival processes such as DNA damage repair, chromatin remodeling, transcription, and cell death signaling. For example, the PARP inhibitor may include, but is not limited to, olaparib, rucaparib, niraparib, iniparib, talazoparib, niraparib, veliparib, fluzoparib, simmiparib, venadaparib, atamparib, pamiparib, stenoparib, 3-aminobenzamide, ME0328, PJ34, AG-14361, INO-1001, UPF-1069, AZD-2461, AZD-5305, AZD-9574, CEP 9722, XAV-939, M2912, DR2313, RBN012759, GeA-69, BYK204165, MN64, RK-287107, 4-hydroxyquinazoline, NMS-P118, picolinamide, NVP-TNKS656, NU1025, WIKI4, G007-LK, 3-aminobenzamide, berberine chloride hydrate, HI-TOPK-032, 4',5,7-trimethoxyflavone, BGP-15 2HCl, or A-966492.

As used herein, the term "PARP inhibitor-resistant cancer" refers to a cancer that has developed resistance to the above-described PARP inhibitor.

As used herein, the term "treatment" refers to intervention aimed at altering the natural processes of individuals or cells having a disease, which may be performed either during the progression of pathological conditions or for prevention thereof. The desired therapeutic effect includes preventing the onset or recurrence of the disease, relieving symptoms, reducing any direct or indirect pathologic consequences of the disease, preventing metastasis, slowing the rate of disease progression, alleviating or temporarily relieving a disease condition, achieving remission, or improving prognosis. In particular, the present disclosure includes any act of ameliorating the progression of cancer by administration of the pharmaceutical composition of the present disclosure. Further, the term "prevention" refers to any act of inhibiting or delaying the onset of cancer by the administration of the composition.

The ADP-ribose binding peptide or pharmaceutically acceptable salt thereof, ADP-ribose, a precursor thereof, or a pharmaceutically acceptable salt thereof included in the pharmaceutical composition may have an amount of 0.0001 to 90 wt%, specifically 0.001 to 50 wt%, and more specifically 0.01 to 20 wt%, based on the total weight of the final composition, respectively, but the amount thereof is not limited thereto.

The pharmaceutical composition may be prepared by further comprising any suitable carrier, excipient, or diluent conventionally used in the preparation of medicaments. Specifically, the pharmaceutical composition of the present disclosure may be formulated and used in the form of oral formulations such as powders, granules, tablets, capsules, suspensions, emulsions, syrups, aerosols, oral patches, and the like, external preparations, external patches, suppositories or sterile injections according to conventional methods, respectively.

When the pharmaceutical composition is used for oral administration, it may be prepared as a sustained-release preparation through appropriate encapsulation, enteric coating, polymer blending, and the like. Further, when the composition is to be used as an injection, various approaches may be considered for formulation, in consideration of the characteristics of the ADP-ribose binding peptide. For example, as described above, the peptide may be modified, such as by PEGylation, binding of fatty acids, or may be coupled to functional moieties via ester linkers, peptide linkers, etc., thereby modulating drug release rates or serum retention time. In addition, the composition may be blended with nanovehicles such as liposomes, polymersomes, micelles, niosome, and dendrimers, or with hydrogels, polymers, etc., to increase drug stability or deliver the drug to the desired site. However, the scope of the present disclosure is not limited to these examples above.

In an embodiment, the sustained-release preparation may be prepared as a long-acting preparation.

In an embodiment, the long-acting formulation may comprise a mixture of polymers and lipids in any suitable ratio, and may further comprise methyl cellulose, albumin, and the like, but is not limited thereto.

The pharmaceutical compositions of the present disclosure may be administered to an individual who has developed or is at risk of developing cancer. As used herein, the term "individual" refers to any animal, including humans.

The pharmaceutical composition of the present disclosure may be administered to a subject in a pharmaceutically effective amount. As used herein, the term "administration" refers to the introduction of a pharmaceutical composition of the present disclosure into a subject by any suitable means, and the route of administration may encompass a variety of routes, including oral or parenteral routes, as long as it reaches the target tissue. Examples of routes of administration may include, but are not limited to, oral, intramuscular, intravenous, arterial, subcutaneous, intraperitoneal, pulmonary, and nasal.

As used herein, the term "pharmaceutically effective amount" means an amount sufficient to prevent and/or treat cancer at a reasonable benefit/risk ratio applicable to a medical use. Suitable dosage amounts and number of doses may be selected according to methods known in the art, and the amount and the number of doses of pharmaceutical composition of the present disclosure actually administered may be suitably determined by a variety of factors, such as the type of symptom to be treated, the route of administration, gender, health status, diet, age of the individual, weight and severity of the disease.

The pharmaceutical composition of the present disclosure may further comprise a third anti-cancer drug.

As used herein, the term "third anti-cancer drug" may be selected from any suitable type for curing, controlling, or alleviating symptoms of cancer, depending on the type of cancer and the extent of its progression, and may be, for example, a cytotoxic anti-cancer drug, a targeted anti-cancer drug, an immune anti-cancer drug, a metabolic anti-cancer drug, a synthetic lethality anti-cancer drug, or a combination thereof.

In the present disclosure, the cytotoxic anti-cancer drug is a drug that exhibits an anti-cancer effect by attacking cancer cells that divide indiscriminately at a faster rate than normal cells, which has the same meaning as that commonly used in the art to which the present disclosure belongs. The cytotoxic anti-cancer drug includes alkylating agents, antimetabolites, and natural anti-cancer drugs.

The alkylating agent may be nitrogen mustards (for example, cyclophosphamide, chlormethine, uramustine, melphalan, chlorambucil, ifosfamide, bendamustine, and the like), alkyl sulfonates (for example, busulfan, procarbazine, and the like), nitrosoureas (for example, carmustine, lomustine, streptozocin, and the like), platinum-based alkylating agents (for example, cisplatin, carboplatin, dicycloplatin, eptaplatin, lobaplatin, myriplatin, nedaplatin, oxaliplatin, picoplatin, satraplatin, triplatin tetranitrate, and the like), but is not limited thereto. The alkylating agent may cause destruction of cancer cells by binding to DNA in cancer cells and damaging the DNA structure.

The antimetabolite may be pyrimidine derivatives (for example, 5-fluorouracil, capecitabine, cytarabine, gemcitabine, fludarabine, and the like), folate derivatives (for example, methotrexate, pemetrexed, and the like), purine derivatives (for example, mercaptopurine, and the like), but is not limited thereto. The antimetabolite may induce cancer cell death by inhibiting metabolism necessary for cell survival and DNA replication.

The natural anti-cancer drug may include topoisomerase inhibitors (camptothecin, epipodophyllotoxin, taxane-based drugs), antibiotics (for example, dactinomycin, doxorubicin, daunorubicin, mitomycin, phleomycin, idarubicin, mitoxantrone HCl, and the like), but are not limited thereto.

In the present disclosure, the targeted anti-cancer drug is an anti-cancer drug that induces the death of cancer cells by inhibiting a target protein (receptor or enzyme) involved in cancer growth, which has the same meaning as that commonly used in the art to which the present disclosure belongs. The targeted anti-cancer drug includes target protein (tyrosine kinase, and the like) inhibitory small molecule compounds and monoclonal antibodies.

In the present disclosure, the targeted anti-cancer drug may be a receptor tyrosine kinase inhibitor targeting at least one target selected from the group consisting of VEGF/VEGFR, EGFR, and HER2.

In an embodiment, the targeted anti-cancer drug usable as the third anti-cancer drug is a VEGF/VEGFR inhibitor. In the present disclosure, examples of the VEGF/VEGFR inhibitor may include monoclonal antibodies such as bevacizumab, ramucirumab, and ranibizumab, together with small molecule compounds such as axitinib, cabozantinib, lapatinib, lenvatinib, pazopanib, regorafenib, sorafenib, sunitinib, and vandetanib, but the VEGF/VEGFR inhibitor is not limited thereto.

In an embodiment, the targeted anti-cancer drug usable as the third anti-cancer drug is an EGFR inhibitor. In the present disclosure, examples of the EGFR inhibitor may include monoclonal antibodies such as cetuximab, panitumumab, zalutumumab, nimotuzumab, and matuzumab together with small molecule compounds such as osimertinib, gefitinib, erlotinib, afatinib, brigatinib, icotinib, and vandetanib, but the EGFR inhibitor is not limited thereto.

In an embodiment, the targeted anti-cancer drug usable as the third anti-cancer drug is a HER2 inhibitor. In the present disclosure, examples of the HER2 inhibitor may include monoclonal antibodies such as trastuzumab, pertuzumab, and margetuximab together with small molecule compounds such as lapatinib, neratinib, and afatinib, but the HER2 inhibitor is not limited thereto.

In addition, examples of the targeted anti-cancer drug of the present disclosure may also include Bcr-Abl targeted anti-cancer drugs such as imatinib, dasatinib, and nilotinib; Src targeted anti-cancer drug such as bosutinib; JAK targeted anti-cancer drugs such as lestaurtinib, ruxolitinib, and pacritinib; MAP2 Kinase targeted anti-cancer drugs such as cobimetinib, selumetinib, trametinib, and binimetinib; MEL4-ALK targeted anti-cancer drugs such as ceritibin and crizotinib, and the like.

In the present disclosure, cancer immunotherapy is a cancer treatment method that activates the body's immune system through an immuno-oncology drug to fight cancer cells. In the present disclosure, the immuno-oncology drug may include immune checkpoint inhibitors, immune cell therapeutic agents, anti-cancer vaccines, and antibody-drug conjugates, wherein the appropriate type thereof may be selected to cure, control, and alleviate symptoms of cancer depending on the type and stage of cancer.

In an embodiment, the immuno-oncology drug may be an immune checkpoint inhibitor, and may be one or more selected from the group consisting of a PD-1 antibody, a PD-L1 antibody, a CTLA-4 antibody, a CD28 antibody, a KIR antibody, a TCR antibody, a LAG-3 antibody, a TIM-3 antibody, a TIGIT antibody, an A2aR antibody, an ICOS antibody, an OX40 antibody, a 4-1BB antibody, and a GITR antibody. For example, the immune checkpoint inhibitor may be PD-1 antibodies such as nivolumab, pembrolizumab, cemiplimab, pidilizumab, toripalimab, and the like; PD-L1 antibodies such as atezolizumab, avelumab, duralumab, and the like; CTLA-4 antibodies such as ipilimumab and tremelimumab, or may be all of these.

In an embodiment, the immuno-oncology drug may be a cell therapeutic agent, and may be a chimeric antigen receptor-natural killer (CAR)-NK agent or a chimeric antigen receptor (CAR)-T agent such as tisagenlecleucel or axicabtagene ciloleucel, but is not limited thereto.

In the present disclosure, the metabolic anti-cancer drug refers to a drug that kills cancer cells by engaging in multiple metabolic processes that are involved in or essential for the growth and survival of cancer cells, such as supplying nutrients to cancer cells, and the like. The metabolic anti-cancer drug may include, for example, IM-156, 3-bromopyruvic acid (3BP), NYH817100, WZB117, GNE-140, AZ93, AZD3965, CPI-613, MKT-077, CB-839, CB-1158, CPI-444, TVB-2640, NDI-010976, TCD-717, ADI-PEG20, Epacadostat, Indoximod, PX478, CPI-0610, RTA402, APO866, GMX1778, AG-221 or AG-120, but is not limited thereto.

As used herein, a synthetic lethality anti-cancer drug refers to an anti-cancer drug that allows cells to survive when only one of two genes (or two gene products) is mutated, but induces synthetic lethality to cause cell death when both genes are mutated. For example, the synthetic lethality-mediated anti-cancer drug may include olaparib, niraparib, talazoparib, veliparib, PRMT5 inhibitors (e.g., TNG908, MRTX1719, AMG 193, PRT811, GSK3326595, PF-06939999, JNJ-64619178, PRT543, PRT811, JBI-778, TNG462, AGX323, AT101, AT201), PRMT1 inhibitors (e.g. GSK3368715, SKL27969), Mat2A inhibitors (e.g., AG-270, IDE397, ISM020), PKMYT1 inhibitors (e.g., RP-6306), PARG inhibitors (e.g., IDE161), etc., but is not limited thereto.

The third anti-cancer drug of the present disclosure may be a combination of one or more cytotoxic anti-cancer drugs, targeted anti-cancer drugs, and/or synthetic lethality anti-cancer drugs, which may be administered at the same time or at different times. Further, the third anti-cancer drug may be at least one selected from the group consisting of dichloroacetic acid, tamoxifen, osimertinib, olaparib, nitrogen mustard, imatinib, oxaliplatin, rituximab, erlotinib, trastuzumab, gefitinib, bortezomib, sunitinib, carboplatin, sorafenib, bevacizumab, cisplatin, cetuximab, viscumalbum, asparaginase, tretinoin, hydroxycarbamide, dasatinib, estramustine, gemtuzumab ozogamicin, ibritumomabtusetan, heptaplatin, methylaminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludagabine, enocitabine, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pirarubicin, aclarubicin, pepromycin, temozolomide, busulfan, ifosfamide, cyclophosphamide, melpharan, altretmine, dacarbazine, thiotepa, nimustine, chlorambucil, mitolactol, lomustine and carmustine, but is not limited thereto.

The ADP-ribose and ADP-ribose binding peptide of the present disclosure may have excellent anti-cancer activity to be prepared not only in pharmaceutical compositions but also in the form of functionalized food compositions.

When the composition of the present disclosure is prepared in the form of a food composition, the food composition may contain additional ingredients that are commonly used in food to improve odor, taste, vision, and the like. For example, food additives may be added. The additives may be selected depending on the type of food and used in an appropriate amount.

The food composition may be prepared as a health functional food, wherein the health functional food is the same term as food for special health use (FoSHU), which refers to food with high medicinal and medical effects processed to efficiently show bioregulatory functions in addition to nutritional supply. The health functional food may be prepared in various forms such as tablets, capsules, powders, granules, liquids, and pills in order to obtain useful effects for the improvement of cancer.

In another general aspect, there is provided a pharmaceutical composition for preventing or treating cancer comprising an ADP-ribose binding peptide or a pharmaceutically acceptable salt thereof as an active ingredient, wherein the ADP-ribose binding peptide is derivatized to have sustained-release properties.

The ADP-ribose binding peptide and derivatization are as described above.

The derivatization of the ADP-ribose binding peptide may comprise, but is not limited to, PEGylation of one or more of its constituent amino acids, or binding of a fatty acid to one or more of the amino acids, thereby having sustained-release properties. The derivatization may be a direct modification of the amino acid or a modification using additional elements such as linkers.

Specifically, the PEG used for the PEGylation may be, but is not limited to, miniPEG2, dPEG4, dPEG6, dPEG8, dPEG12, dPEG24, or the like.

Specifically, the fatty acid may include, but are not limited to, butyric acid, caproic acid, caprylic acid, capric acid, lauric acid, myristic acid, palmitic acid, stearic acid, arachidic acid, behenic acid, lignoceric acid, cerotic acid, montanic acid, melissic acid, corynomycolic acid, etc.

In one specific embodiment of the present disclosure, an ADP-ribose binding peptide derivative was prepared by combining miniPEG2, a cell-penetrating peptide, an ADP-ribose binding peptide and a fatty acid, and in particular, an derivative prepared by PEGylating the peptide of SEQ ID NO: 29 (GRKKRRQRRRPQFLYVADLENMVQYRRNEHGRRRKIKR) and binding a fatty acid thereto is termed a "sustained release derivative". The specific structure of the sustained release derivative of the present disclosure is shown in the following Chemical Formula 3:

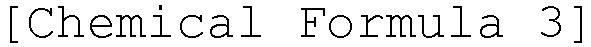

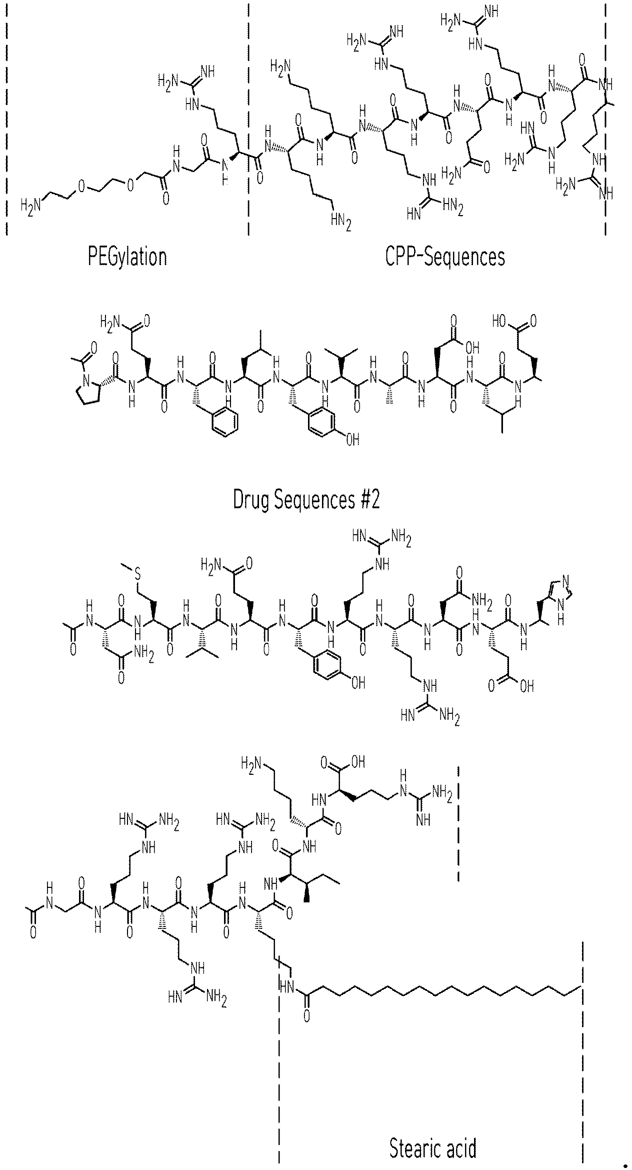

In another aspect of the present disclosure, derivatives prepared by PEGylating the peptide of SEQ ID NOs: 1 to 14 and binding a fatty acid thereto and an additional cell-penetrating have been found to be as effective as the "sustained release derivative" prepared by using the peptide of SEQ ID NO: 29 above.

Exemplary embodiments of the present disclosure may be modified in various other forms, and the scope of the present disclosure is not limited to the exemplary embodiments to be described below. In addition, the exemplary embodiments of the present disclosure are provided to more completely explain the present disclosure to an ordinary person skilled in the art. Further, "including" a component throughout the specification does not mean excluding other components, but rather it means that other components may be further included, unless otherwise stated.

### [Mode for Carrying out the Invention]

Hereinafter, the present disclosure will be described in more detail through Examples and Experimental Examples. However, the following Examples and Experimental Examples are provided only for illustrating the present disclosure, and the scope of the present disclosure is not limited thereto.

### Examples 1 to 29. Preparation of adenosine diphosphate (ADP)-ribose binding peptide

ADP-ribose binding peptides of Examples 1 to 14 were synthesized from WWE domains present in various types of proteins and used in the experiments. The specific information of the peptides used is shown in Table 1 below.

**[Table 1]**

| **Examples** | **Sequence Information (SEQ ID NO)** | **Derived Proteins** |
|---|---|---|
| 1 | RRYTVQFTTMVQVNEETGNRRPVM (SEQ ID NO: 1) | HUWE1 |
| 2 | FLYVADLENMVQYRRNEHGRRRKIKR (SEQ ID NO: 2) | RNF146 |
| 3 | RVYTIDFNSMQQINEDTGTARAIQR (SEQ ID NO: 3) | TRIP12 |
| 4 | VPYIIDLQSMHQFRQDTGTMRPVRR (SEQ ID NO: 4) | DTX1 |
| 5 | FSYKIDFAEMKQMNLTTGKQRLIKR (SEQ ID NO: 5) | PARP11 |
| 6 | GRYDVHLGERMRYAVYWDELASEVRR (SEQ ID NO: 6) | DDHD2 |
| 7 | APYIIDLQSMNQFRQDTGTLRPVRR (SEQ ID NO: 7) | DTX4 |
| 8 | APYIIDLPSWTQFRQDTGTMRAVRR (SEQ ID NO: 8) | DTX2 |
| 9 | FCYLIYFNSMSQMNRQTRRRRRLRR (SEQ ID NO: 9) | PARP7 |
| 10 | FSYVIDFNTMGQINRQTQRQRRVRR (SEQ ID NO: 10) | PARP12 |
| 11 | YNYTVNYTTHTQTNKTSSFCRSVRR (SEQ ID NO: 11) | PIK3C2G |
| 12 | WIWYWKNESGTWIQYGEEKDKREN (SEQ ID NO: 12) | ZC3HAV1 |
| 13 | RHYTVNLNTYTATDTKGHSLSVQR (SEQ ID NO: 13) | PARPI4 |
| 14 | TAYEASVCDYLEQQVARGN (SEQ ID NO: 14) | HNRNPA2B1 |

Further, peptides of Examples 15 to 28 were prepared by conjugating a cell-penetrating peptide to the N-terminus of the peptides of Examples 1 to 14 above. The peptide sequences of Examples 15 to 28 are shown in Table 2 below.

**[Table 2]**

| **Examples** | **Sequence Information (SEQ ID NO)** |
|---|---|
| 15 | GRKKRRQRRRPQRRYTVQFTTMVQTNEETGNRRPVM (SEQ ID NO: 15) |
| 16 | RAGLQFPVGRLLRRLLRFLYVADLENMVQYRRNEHGRRREIKR (SEQ ID NO: 16) |
| 17 | GRKKRRQRRRPQRVYTIDFNSMQQINEDTGTARAIQR (SEQ ID NO: 17) |
| 18 | GRKKRRQRRRTPQVPYIIDLQSMHQFRQDTGTRPVRR (SEQ ID NO: 18) |
| 19 | GRKKRRQRRRPQFSYKIDFAEMKQMNLTTGRQRLIKR (SEQ ID NO: 19) |
| 20 | GRKKRRQRRRPQGRYDVHLGERMRYAVYWDELASEVRR (SEQ ID NO: 20) |
| 21 | GRKKRRQRRRPQAPYIIDLQSMNQFRQDTGTLRPVRR (SEQ ID NO: 21) |
| 22 | GRKKRRQRRRPQAPYIIDLPSWTQFRODTGTMRAVRR (SEQ ID NO: 22) |
| 23 | GRKKRRQRRRPQFCYLIYFNSMSQMNRQTRRRRRLRR (SEQ ID NO: 23) |
| 24 | GRKKRRQRRRPQFSYVIDFNTMGQINRQTQRQRRVRR (SEQ ID NO: 24) |
| 25 | GRKKRRQRRRPQYNYTVNYTTHTQTNKTSSFCRSVRR (SEQ ID NO: 25) |
| 26 | GRKKRRQRRRPQWIWYWKNESGTWIQYGEEKDKRKN (SEQ ID NO: 26) |
| 27 | GRKKRRQRRRPQRHYTVNLNTYTATDTKGHSLSVQR (SEQ ID NO: 27) |
| 28 | GRKKRRQRRRPQTAYEASVCDYLEQQVARGN (SEQ ID NO: 28) |

In addition, the peptide of Example 29 was prepared by conjugating a cell-penetrating peptide (GRKKRRQRRRPQ) to the N-terminus of the peptide of Example 2 above. The peptide sequences of Example 29 are shown in Table 3 below.

**[Table 3]**

| **Example** | **Sequence Information (SEQ ID NO)** |
|---|---|
| 29 | GRKKRRQRRRPQFLYVADLENMVQYRRNEHGRRRKIKR (SEQ ID NO: 29) |

The change in cancer cell viability was measured by treating the above-prepared peptides of Example 1 to 15 and 17 to 29. In Aspc-1 cells (FIG. 1, left) and MDA-MB-231 cells (FIG. 1, right), the anti-cancer effect of all peptides of Examples alone could be confirmed, and the GI50 (half maximal growth inhibition concentration) of each Example was confirmed.

### Example 30. Preparation of sustained release derivative using peptide of Example 29

A derivative of the ADP-ribose binding peptide synthesized by PEGylating the peptide of Example 29 and binding a stearic acid thereto, was commissioned from Peptron. This derivative, named a "sustained release derivative", was then subjected to mass spectrometry (SHIMADZU LCMS-2020 System) and HPLC analysis (SHIMADZU Prominence HPLC System) .

For HPLC analysis, the column was a Shiseido capcell pak C18, 120 Å (4.6 x 50 mm), 5 µm, and the column temperature was maintained at room temperature. The flow rate was 1.0 mL/min, and detection was performed at 220 nm. Mobile phase A was 0.1% trifluoroacetic acid (TFA) in water and mobile phase B was 0.1% TFA in acetonitrile. The gradient was 35 %B for 2 minutes, 30 %B for 10 minutes, 10 %B for 1 minute, and 77 %B for 4 minutes.

The results are shown in Table 4 below, where the LC-MS [M+H]⁺ value was found to be 5363.43.

**[Table 4]**

| **Detector A - 1 (220nm)** | | | | | |
|---|---|---|---|---|---|
| **Pk #** | **Retention Time** | **Area** | **Area %** | **Height** | **Height %** |
| 1 | 5.217 | 9350 | 0.401 | 1640 | 0.581 |
| 2 | 5.375 | 2235 | 0.096 | 479 | 0.170 |
| 3 | 5.508 | 4758 | 0.204 | 1002 | 0.355 |
| 4 | 6.325 | 2279816 | 97.658 | 273458 | 96.903 |
| 5 | 7.242 | 7468 | 0.320 | 1206 | 0.427 |
| 6 | 7.567 | 19001 | 0.814 | 3026 | 1.072 |
| 7 | 13.475 | 11857 | 0.508 | 1387 | 0.491 |
| **Totals** | | **2334485** | **100.000** | **282198** | **100.000** |

### Experimental Example 1. Changes in cancer cell viability by combination treatment of peptides according to Examples and ADP-ribose

### Experimental Example 1-1. Changes in Caki-1 cell viability by combination treatment of peptides according to Examples and ADP-ribose

2.5 X 10³ Kidney cancer (Caki-1) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 1 at 25 µM concentration alone
4) Group treated with each CPP-peptide of Example 15 at 10 µM concentration alone
5) Group treated in combination with ADPR at 1 mM concentration and peptide of Example 1 at 25 µM concentration (Combination 1)
6) Group treated in combination with ADPR at 1 mM concentration and CPP-peptide of Example 15 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that Caki-1 cells were growing rapidly in the peptide untreated group, while the viability was reduced in all the groups treated with peptides of Examples of the present disclosure and ADPR. In particular, in the group treated in combination with the peptides of Examples and ADPR, Caki-1 cells were significantly inhibited in growth, with observable signs of cell death (FIG. 2).

### Experimental Example 1-2. Changes in HCC1937 cell viability by combination treatment of peptides according to Examples and ADP-ribose

2.5 X 10³ Breast cancer (HCC1937) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 2 at 25 µM concentration alone
4) Group treated with each CPP-peptide of Example 16 at 10 µM concentration alone
5) Group treated in combination with ADPR at 1 mM concentration and peptide of Example 2 at 25 µM concentration (Combination 1)
6) Group treated in combination with ADPR at 1 mM concentration and CPP-peptide of Example 16 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that HCC1937 cells were growing rapidly in the peptide untreated group, while the viability was reduced in all the groups treated with peptides of Examples of the present disclosure and ADPR. In particular, in the group treated in combination with the peptides of Examples and ADPR, HCC1937 cells were significantly inhibited in growth, with observable signs of cell death (FIG. 3).

### Experimental Example 1-3. Changes in AsPC-1 cell viability by combination treatment of peptides according to Examples and ADP-ribose

2.5 × 10³ Pancreatic cancer (AsPC-1) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 3 at 25 µM concentration alone
4) Group treated with each CPP-peptide of Example 17 at 10 µM concentration alone
5) Group treated in combination with ADPR at 1 mM concentration and peptide of Example 3 at 25 µM concentration (Combination 1)
6) Group treated in combination with ADPR at 1 mM concentration and CPP-peptide of Example 17 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that AsPC-1 cells were growing rapidly in the peptide untreated group, while the viability was reduced in all the groups treated with peptides of Examples of the present disclosure and ADPR. In particular, in the group treated with the combination of the peptides of Examples and ADPR, AsPC-1 cells were significantly inhibited in growth, with observable signs of cell death (FIG. 4).

### Experimental Example 1-4. Changes in HepG2 cell viability by combination treatment of peptides according to Examples and ADP-ribose

2.5 × 10³ Liver cancer (HepG2) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 4 at 25 µM concentration alone
4) Group treated with each CPP-peptide of Example 18 at 10 µM concentration alone
5) Group treated in combination with ADPR at 1 mM concentration and peptide of Example 4 at 25 µM concentration (Combination 1)
6) Group treated in combination with ADPR at 1 mM concentration and CPP-peptide of Example 18 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that HepG2 cells were growing rapidly in the peptide untreated group, while the viability was reduced in all the groups treated with peptides of Examples of the present disclosure and ADPR. In particular, in the group treated in combination with the peptides of Examples and ADPR, HepG2 cells were significantly inhibited in growth, with observable signs of cell death (FIG. 5).

### Experimental Example 1-5. Changes in H1975 cell viability by combination treatment of peptides according to Examples and ADP-ribose

2.5 × 10³ Lung cancer (H1975) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 5 at 25 µM concentration alone
4) Group treated with each CPP-peptide of Example 19 at 10 µM concentration alone
5) Group treated in combination with ADPR at 1 mM concentration and peptide of Example 5 at 25 µM concentration (Combination 1)
6) Group treated in combination with ADPR at 1 mM concentration and CPP-peptide of Example 19 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that H1975 cells were growing rapidly in the peptide untreated group, while the viability was reduced in all the groups treated with peptides of Examples of the present disclosure and ADPR. In particular, in the group treated in combination with the peptides of Examples and ADPR, H1975 cells were significantly inhibited in growth, with observable signs of cell death (FIG. 6).

### Experimental Example 1-6. Changes in HCT116 cell viability by combination treatment of peptides according to Examples and ADP-ribose

2.5 × 10³ Colon cancer (HCT116) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with ADPR at 1 mM concentration alone
3) Group treated with peptide of Example 6 at 25 µM concentration alone
4) Group treated with each CPP-peptide of Example 20 at 10 µM concentration alone
5) Group treated in combination with ADPR at 1 mM concentration and peptide of Example 6 at 25 µM concentration (Combination 1)
6) Group treated in combination with ADPR at 1 mM concentration and CPP-peptide of Example 20 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, it was found that HCT116 cells were growing rapidly in the peptide untreated group, while the viability was reduced in all the groups treated with peptides of Examples of the present disclosure and ADPR. In particular, in the group treated in combination with the peptides of Examples and ADPR, HCT116 cells were significantly inhibited in growth, with observable signs of cell death (FIG. 7).

### Experimental Example 2. Confirmation of normal cytotoxicity upon combination treatment of peptides according to Examples and ADP-ribose

In a 96-well plate, 2.5 × 10³ human-derived normal CCD-18-Co or MRC5 cells were cultured for 24 hours under 37°C and 5% CO₂ conditions, and then treated in combination with the peptides of Examples 1 to 15 and 17 to 29 and ADP-ribose.

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The MTT reagent was removed at the end of the reaction, 200 µl of dimethyl sulfoxide was then added to each well, and the absorbance was measured to determine the cell viability.

As a result, it was found that normal CCD-18-Co cells survived by approximately 90% or more when treated in combination with each peptide of Examples and ADPR as compared to the peptide untreated group. Thus, it was found that combination treatment of each peptide of Examples and ADPR showed little toxicity to normal cells (FIG. 8).

### Experimental Example 3. Comparison and verification of anti-cancer efficacy by combination treatment of peptides of Examples and ADP-ribose using animal models

### Experimental Example 3-1. Changes in tumor volume by combination treatment of peptides according to Examples and ADP-ribose in AsPC-1 cells

Pancreatic cancer (AsPC-1) cells (1 × 10⁷) were inoculated into 5-week-old BALB/c nude mice on the back flank, and the mice were divided into the following treatment groups and treated with each peptide according to Examples at GI50 concentration alone or in combination.
1) Untreated group
2) Group treated with ADP-ribose alone
3) Group treated with each peptide of Examples 1 to 6 alone
4) Groups treated in combination with ADPR and each peptide of Examples 1 to 6 (Combinations 1 to 6).

When the tumor volume reached about 75 mm³, ADP-ribose and the peptides of Examples 1 to 6 were administered alone or in combination. Here, ADP-ribose at a concentration of 10 mg/kg and the peptides of Examples 1 to 6 at a concentration of 20 mg/kg were administered by subcutaneous route twice weekly for 4 weeks.

Tumor size was then measured using a digital caliper and the results of the change in tumor volume were compared between groups.

The results showed that tumor volume was significantly reduced in all treatment groups compared to the control group. In particular, the combination treatment group showed a significant reduction in tumor volume compared to the single treatment group (FIGS. 9 and 10).

### Experimental Example 3-2. Changes in tumor volume by combination treatment of CPP-peptides according to Examples and ADP-ribose in HCC1937 cells

Breast cancer (HCC1937) cells (1 × 10⁷) were inoculated into 5-week-old BALB/c nude mice on the back flank, and the mice were divided into the following treatment groups and treated with each CPP-peptide according to Examples at GI50 concentration alone or in combination.
1) Untreated group
2) Group treated with ADP-ribose alone
3) Group treated with each CPP-peptide of Examples 15 to 20 alone
4) Groups treated in combination with ADPR and each CPP-peptide of Examples 15 to 20 (Combinations A to F).

When the tumor volume reached about 75 mm³, ADP-ribose and the CPP-peptides of Examples 15 to 20 were administered alone or in combination. Here, ADP-ribose at a concentration of 10 mg/kg and the CPP-peptides of Examples 15 to 20 at a concentration of 10 mg/kg were administered by subcutaneous route twice weekly for 4 weeks.

Tumor size was then measured using a digital caliper and the results of the change in tumor volume were compared between groups.

The results showed that tumor volume was significantly reduced in all treatment groups compared to the control group. In particular, the combination treatment group showed a significant reduction in tumor volume compared to the single treatment group (FIGS. 11 and 12).

### Experimental Example 4. Confirmation of anti-cancer synergistic effects of combination treatment of peptides according to Examples and ADP-ribose in PARP-resistant cell lines

Next, the effect of combination treatment of the peptide of Examples with ADP-ribose was confirmed in a PARP inhibitor-resistant cancer cell line.

Specifically, 2.5 × 10³ PARP inhibitor-resistant breast cancer (HCC1937) cells were cultured in a 96-well plate for 24 hours under 37°C and 5% CO₂ conditions.

The cells were then divided into the following groups and treated with each peptide according to Examples at GI50 concentration alone or in combination.
1) Untreated group
2) Group treated with PARP inhibitor (Olaparib) at 2 µM concentration
3) Group treated with ADPR at 1 mM concentration alone
4) Groups treated with each peptide of Examples 1 to 6 at 25 µM concentration alone
5) Groups treated with each CPP-peptide of Examples 15 to 20 at 10 µM concentration alone
6) Group treated in combination with ADPR at 1 mM concentration and each peptide of Examples 1 to 6 at 25 µM concentration (Combination 1)
6) Group treated in combination with ADPR at 1 mM concentration and CPP-peptide of Examples 15 to 20 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

The results showed that when HCC1937 cells were treated with Olaparib, a PARP inhibitor, alone, approximately 80% of the cancer cells were viable, but treatment with the peptides of Examples or ADP-ribose significantly reduced cell viability in all cases. In particular, all cases of combination treatment with the peptides according to Examples and ADP-ribose showed a significant reduction in cancer cell viability compared to the single treatment group (FIG. 13).

### Experimental Example 5. Changes in cancer cell viability by combination treatment of peptides according to Examples and NAD⁺

Considering the possibility that NAD⁺, a precursor of ADP-ribose, could replace ADP-ribose, the present inventors confirmed its anti-cancer effects when administered with NAD⁺ alone and in combination with ADP-ribose binding peptides according to Examples.

Specifically, 2.5 × 10³ ovarian cancer (OVCAR-3) cells were cultured in a 96-well plate for 24 hours under 37°C and 5% CO₂ conditions, and then divided into the following groups and treated with each peptide according to Examples at GI50 concentration alone or in combination.
1) Untreated group
2) Group treated with NAD⁺ at 1 mM concentration alone
3) Group treated with peptide of Example 2 at 25 µM concentration alone
4) Group treated with CPP-peptide of Example 16 at 10 µM concentration alone
5) Group treated in combination with NAD⁺ at 1 mM concentration and peptide of Example 2 at 25 µM concentration (Combination 1)
6) Group treated in combination with NAD⁺ at 1 mM concentration and CPP-peptide of Example 16 at 10 µM concentration (Combination 2)

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

The results showed that the peptide untreated group had dramatic growth of OVCAR-3 cells, and the NAD⁺ treatment alone group was not significantly different from the control group. However, the combination treatment of the peptides of Examples according to the present disclosure and NAD⁺ significantly reduced the viability of cancer cells compared to the treatment of the peptides of Examples alone, indicating a superior anti-cancer effect (FIG. 14).

### Experimental Example 6. Changes in cancer cell viability by combination treatment of peptides according to Examples, ADP-ribose, NAD⁺, and various anti-cancer drugs

Each of 2.5 × 10³ human-derived pancreatic cancer (Aspc-1), kidney cancer (Caki-1), breast cancer (HCC1937), liver cancer (HepG2), lung cancer (H1975), colon cancer (HCT116), ovarian cancer (OVCAR-3), brain cancer (U87) cells were cultured in a 96-well plate under 37°C and 5% CO₂ conditions for 24 hours, and then divided into the following groups and treated with each formulation at GI20/IC20 concentrations alone or in combination.
1) Untreated group
2) Group in which each cancer cell was treated with peptides of Examples 1 to 14 at 25 µM concentration and Examples 15, 17 to 29 at 10 µM concentration, respectively, alone.
3) Group treated with ADPR at 2 mM concentration alone
4) Group treated with NAD⁺ at 2 mM concentration alone
5) Groups treated with various anti-cancer drugs alone (Cisplatin at 2 µM, Docetaxel at 1 µM, Dichloroacetate at 3 mM, Doxorubicin at 2 µM, Sorafenib at 2 µM, Osimertinib at 10 nM, Trastuzumab at 0.2 µM, Bevacizumab at 0.2 nM, Tamoxifen at 0.2 µM, or Olaparib at 1 µM)
6) Groups treated in combination with ADPR at 2 mM concentration and each peptide according to Examples (Examples 1 to 14 at 25 µM, and Examples 15, 17 to 29 at 10 µM).
7) Groups treated in combination with ADPR at 2 mM concentration, NAD⁺ at 2 mM concentration, and each peptide according to Examples (Examples 1 to 14 at 25 µM, and Examples 15, 17 to 29 at 10 µM).
8) Groups treated in combination with ADPR at 2 mM concentration, peptides according to Examples (Examples 1 to 14 at 25 µM, and Examples 15, 17 to 29 at 10 µM), and various anti-cancer drugs (same concentration as Group 5 above), respectively.

The cells were then cultured for another 96 hours under 37°C and 5% CO₂ conditions, and 10 µl of 3-(4,5-dimethylthiazol-2-yl)-2,5-diphenyltetrazolium bromide reagent was added to each well and reacted for 1 hour. The MTT reagent was removed at the end of the reaction, 200 µl of dimethyl sulfoxide was then added to each well, and the absorbance was measured to determine the cell viability.

As a result, in all cancer cells used in the experiment, compared to treatment with the peptides of Examples 1 to 15 and 17 to 29, ADPR, NAD⁺, and various anti-cancer drugs alone, the combination treatment of each peptide according to Examples and ADPR resulted in about 40% cancer cell viability, and the combination treatment of each peptide according to Examples, ADPR, and NAD⁺ resulted in about 20% cancer cell viability. Further, the combination treatment of the peptides according to Examples 1 to 15 and 17 to 29, ADPR, and various anti-cancer drugs significantly reduced the viability of cancer cells compared to all other groups, resulting in a viability of about 10%. Thus, it was found that the combination treatment of the peptides according to Examples, ADPR, and various anti-cancer drugs resulted in excellent anti-cancer effects (FIGS. 15 to 28).

### Experimental Example 7. Changes in cancer cell viability upon treatment with sustained release derivative prepared using the peptide of Example 29, alone or in combination

Each of 2.5 × 10³ human-derived pancreatic cancer (Aspc-1), kidney cancer (Caki-1), breast cancer (HCC1937), liver cancer (HepG2), lung cancer (H1975), colon cancer (HCT116), ovarian cancer (OVCAR-3), brain cancer (U87) cells were cultured in a 96-well plate under 37°C and 5% CO₂ conditions for 24 hours, and then treated with the sustained release derivative alone. In addition, MDA-MB-231 cancer cells were divided into the following groups and treated with each formulation at GI20 concentration alone or in combination.
1) Untreated group
2) Group treated with sustained-release derivative alone at 5 µM concentration
3) Group treated with ADPR at 2 mM concentration alone
4) Group treated with NAD⁺ at 2 mM concentration alone
5) Groups treated with various anti-cancer drugs alone (Cisplatin at 2 µM, Docetaxel at 1 µM, Dichloroacetate at 3 mM, Doxorubicin at 2 µM, Sorafenib at 2 µM, Osimertinib at 10 nM, Trastuzumab at 0.2 µM, Bevacizumab at 0.2 nM, Tamoxifen at 0.2 µM, or Olaparib at 1 µM)
6) Group treated in combination with ADPR at 2 mM concentration and a sustained-release derivative at 5 µM concentration
7) Group treated in combination with ADPR at 2 mM concentration, NAD⁺ at 2 mM concentration, and a sustained-release derivative at 5 µM concentration
8) Group treated in combination with ADPR at 2 mM concentration, a sustained-release derivative at 5 µM concentration, and various anti-cancer drugs (same concentration as Group 5 above).

The cells were then cultured for another 96 hours at 37°C and 5% CO₂, and 10 µl of 3-(4,5-dimethylthiazol-2-yl)-2,5-diphenyltetrazolium bromide reagent was added to each well and reacted for 1 hour. The reagent was removed at the end of the reaction, 200 µl of dimethyl sulfoxide was then added to each well, and the absorbance was measured to determine the cell viability.

As a result, treatment with the sustained-release derivative alone resulted in viability of less than 10% for most cancer cell lines (FIG. 29, left). Meanwhile, MDA-MB-231, a triple-negative breast cancer cell line, showed drug resistance with viability of 80% when treated with the sustained-release derivative, ADPR, NAD⁺, or various anti-cancer drugs alone, but showed cytotoxicity when combined with sustained-release derivative and ADPR, and showed significantly superior cytotoxicity at the time of triple-drug combination treatment with NAD⁺ or various anti-cancer drugs (FIG. 29, right).

### Experimental Example 8. Confirmation of normal cytotoxicity upon treatment with sustained release derivative prepared using the peptide of Example 29, alone or in combination

In a 96-well plate, 2.5 × 10³ human-derived normal MRC5 cells were cultured for 24 hours under 37°C and 5% CO₂ conditions, and then treated with the sustained-release derivative and ADPR alone or in combination.

The cells were then cultured for another 96 hours at 37°C and 5% CO₂, and 10 µl of 3-(4,5-dimethylthiazol-2-yl)-2,5-diphenyltetrazolium bromide reagent was added to each well and reacted for 1 hour. The MTT reagent was removed at the end of the reaction, 200 µl of dimethyl sulfoxide was then added to each well, and the absorbance was measured to determine the cell viability.

The results showed that compared to the untreated group, treatment with the sustained-release derivative alone or in combination with ADPR resulted in approximately 90% or more viability of normal MRC5 cells. Thus, it was found that treatment with the sustained release derivative exhibited little toxicity to normal cells, regardless of whether the sustained release derivative was used alone or in combination (FIG. 30).

### Experimental Example 9. Comparison and verification of anti-cancer efficacy of sustained-release derivative alone or in combination treatment with immunotherapeutic agent using animal models

Colon cancer (MC38) cells (1 × 10⁶/100 µL) were inoculated into 6-week-old male C57BL/6-hPD1 (PD-1 humanized mice) on the right dorsal forelimb area of mice, and the treatment groups were divided as follows.
1) Untreated group
2) Group treated with sustained-release derivative alone at GI50 concentration by intravenous injection
3) Group treated with sustained-release derivative alone at GI50 concentration by subcutaneous injection
4) Group treated with immunotherapeutic agent Nivolumab alone at GI50 concentration by intraperitoneal injection
5) Group treated in combination with sustained-release derivative at GI50 concentration (intravenous injection) and Nivolumab at GI50 concentration (intraperitoneal injection) (Combination 1)
6) Group treated in combination with sustained-release derivative at GI50 concentration (subcutaneous injection) and Nivolumab at GI50 concentration (intraperitoneal injection) (Combination 2)

When the tumor volume reached approximately 100 mm³, the sustained-release derivative or Nivolumab was administered alone or in combination. Here, the sustained-release derivative at a concentration of 5 mg/kg was mixed with albumin in a 3:1 ratio, and Nivolumab was used at a concentration of 5 mg/kg, each administered twice weekly for 4 weeks.

Tumor size was then measured using a digital caliper and the results of the change in tumor volume were compared between groups.

The results showed that tumor volume was significantly reduced in all treatment groups compared to the control group. In particular, the combination treatment group showed a significant reduction in tumor volume compared to the single treatment group (FIG. 31). Further, no change in body weight was observed in all treatment groups, confirming the absence of toxicity (FIG. 32).

From the above description, those skilled in the art to which the present disclosure pertains will understand that the present disclosure may be embodied in other specific forms without changing the technical spirit or essential characteristics thereof. In this regard, it should be understood that the embodiments described above are illustrative in all respects and not restrictive. The scope of the present disclosure is indicated by the following claims rather than the detailed description, and should be construed as including all changes or modifications derived from the meaning and scope of the claims and equivalent concepts within the scope of the present disclosure.

## Claims

1. A pharmaceutical composition for preventing or treating cancer comprising:
a) an adenosine diphosphate (ADP)-ribose binding peptide or a pharmaceutically acceptable salt thereof; and
b) ADP-ribose, a precursor thereof, or a pharmaceutically acceptable salt thereof.

2. The pharmaceutical composition for preventing or treating cancer of claim 1, wherein the ADP-ribose binding peptide consists of any one amino acid sequence selected from the group consisting of SEQ ID NOs: 1 to 14.

3. The pharmaceutical composition for preventing or treating cancer of claim 1, wherein the precursor of the ADP-ribose is nicotinamide adenine dinucleotide (NAD⁺), cADPR, poly-ADPR, nicotinic acid, nicotinamide, or nicotinamide riboside.

4. The pharmaceutical composition for preventing or treating cancer of claim 1, wherein the peptide further comprises a cell-penetrating peptide at the N-terminus, C-terminus, or both termini.

5. The pharmaceutical composition for preventing or treating cancer of claim 4, wherein the cell-penetrating peptide is at least one selected from the group consisting of TAT, buforin, maurocalcine, penetratin, poly-arginine-derived peptides, Antennapedia, Transportan, VP22, Hph-1, poly-arginine R11(R9), Pep-1, HP4, LAH4, Vetofusin-1, signal sequence-based peptides, and amphipathic peptides.

6. The pharmaceutical composition for preventing or treating cancer of claim 4, wherein the peptide comprises any one amino acid sequence selected from the group consisting of SEQ ID NOs: 15 to 29.

7. The pharmaceutical composition for preventing or treating cancer of claim 1, wherein the peptide comprises one or more of its constituent amino acids that are acetylated, hydroxylated, methylated, amidated or PEGylated.

8. The pharmaceutical composition for preventing or treating cancer of claim 1, wherein the peptide comprises a fatty acid, carbohydrate, lipid component, or cofactor bound to one or more of its constituent amino acids.

9. The pharmaceutical composition for preventing or treating cancer of claim 1, wherein the cancer is at least one solid cancer selected from the group consisting of brain cancer, head and neck cancer, lung cancer, breast cancer, thymoma, esophageal cancer, colon cancer, liver cancer, stomach cancer, pancreatic cancer, biliary tract cancer, kidney cancer, bladder cancer, prostate cancer, testicular cancer, germ cell tumor, ovarian cancer, cervical cancer, endometrial cancer, colorectal cancer, lymphoma, acute leukemia, chronic leukemia, multiple myeloma, sarcoma, malignant melanoma, and skin cancer.

10. The pharmaceutical composition for preventing or treating cancer of claim 1, wherein the cancer is a poly (ADP-ribose) polymerase (PARP) inhibitor-resistant cancer.

11. The pharmaceutical composition for preventing or treating cancer of claim 1, wherein the pharmaceutical composition is formulated as a sustained-release preparation.

12. The pharmaceutical composition for preventing or treating cancer of claim 1, further comprising a third anti-cancer drug.

13. The pharmaceutical composition for preventing or treating cancer of claim 12, wherein the third anti-cancer drug is a cytotoxic anti-cancer drug, a targeted anti-cancer drug, an immune anti-cancer drug, a metabolic anti-cancer drug, a synthetic lethality anti-cancer drug, or a combination thereof.

14. A pharmaceutical composition for preventing or treating cancer, comprising an ADP-ribose binding peptide or a pharmaceutically acceptable salt thereof as an active ingredient, wherein the ADP-ribose binding peptide is derivatized to have sustained-release properties.

15. The pharmaceutical composition for preventing or treating cancer of claim 14, wherein the derivatization of the ADP-ribose binding peptide comprises PEGylation of one or more of its constituent amino acids, or binding of a fatty acid to one or more of its constituent amino acids.

16. The pharmaceutical composition for preventing or treating cancer of claim 14, wherein the derivatized ADP-ribose binding peptide is represented by the following Chemical Formula 3:
